(19) Europäisches Patentamt
European Patent Office
Office européen des brevets

(11) EP 2 727 604 A1

(12) **EUROPEAN PATENT APPLICATION**
published in accordance with Art. 153(4) EPC

(43) Date of publication:
**07.05.2014 Bulletin 2014/19**

(21) Application number: **12805180.2**

(22) Date of filing: **29.06.2012**

(51) Int Cl.:
***A61K 45/00*** *(2006.01)* ***A61K 31/439*** *(2006.01)*
***A61K 31/444*** *(2006.01)* ***A61K 31/5377*** *(2006.01)*
***A61P 17/04*** *(2006.01)* ***A61P 43/00*** *(2006.01)*
***A61K 31/4427*** *(2006.01)* ***A61K 31/501*** *(2006.01)*
***A61K 31/551*** *(2006.01)*

(86) International application number:
**PCT/JP2012/066675**

(87) International publication number:
**WO 2013/002365 (03.01.2013 Gazette 2013/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.06.2011 JP 2011145447**

(71) Applicant: **Toray Industries, Inc.**
**Tokyo 103-8666 (JP)**

(72) Inventor: **HAYASHI, Kenichi**
**Kanagawa**
**248-8555 (JP)**

(74) Representative: **Kador & Partner**
**Corneliusstrasse 15**
**80469 München (DE)**

# (54) ANTIPRURITIC AGENT

(57) An object of the present invention is to provide an antipruritic which exerts an antipruritic effect based on a novel action mechanism and is effective for pruritus. The present invention provides an antipruritic containing as an effective ingredient a compound which activates a central type nicotinic acetylcholine receptor.

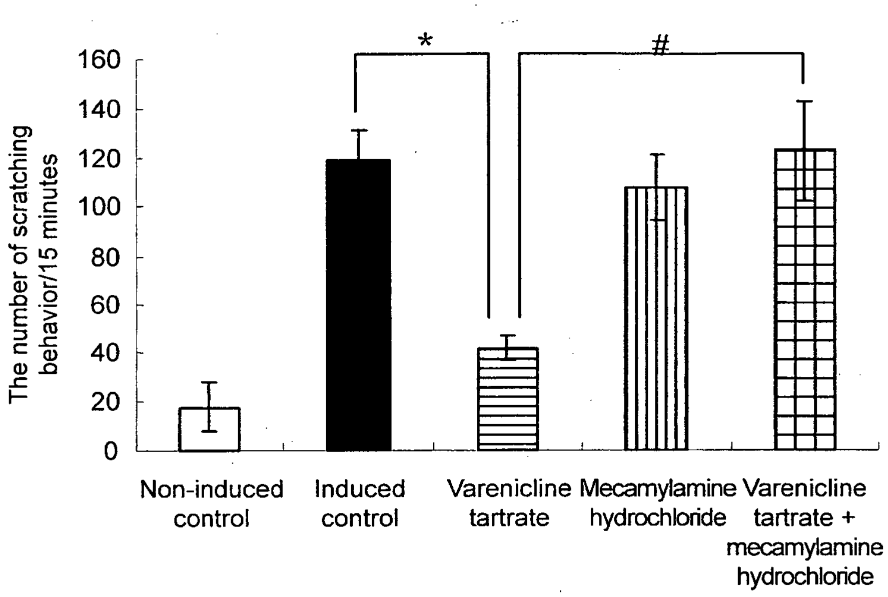


**Fig.1**

EP 2 727 604 A1

## Description

### TECHNICAL FIELD

The present invention relates to an antipruritic.

### BACKGROUND ART

Pruritus is a sensation peculiar to skin and is caused by skin diseases in many cases. However, it may be caused by certain types of internal diseases (malignant tumor, diabetes, hepatic diseases, chronic renal diseases, renal failure, gout, thyroid diseases, hematological disorders and iron deficiency) as well as hemodialysis, peritoneal dialysis, pregnancy, parasitic infection and multiple sclerosis; or may also be caused by drug allergy (drug-induced pruritus) or mental stress (psychogenic pruritus).

Although the mechanism which induces pruritus has not yet been completely clarified, it is believed that, in the body, an endogenous stimulant such as histamine, substance P, bradykinin, proteinase, prostaglandin or opioid peptide acts on multiple stimuli-responsive nerve endings (pruritus receptors) that are present in dermoepidermal junctions, and generated impulses are transmitted to spinothalamic tract, thalamus and cerebral cortex in this order, thereby generating the sensation of pruritus (Non-patent Document 1). Further, although pruritus was considered to be weak pain, it has become clear that pruritus and pain are sensations that are transmitted through different neural pathways and generated by different mechanisms (Non-patent Documents 2 and 3).

For the treatment of pruritus, antihistamines are mainly used as oral drugs and opioid $\kappa$ receptor agonists may also be used for hemodialysis patients. As external preparations, antihistamines, corticosteroids, immunosuppressants or non-steroidal antiinflammatory agents are used.

On the other hand, nicotinic acetylcholine receptors are distributed widely throughout central and peripheral tissues, which are homopentamers or heteropentamers composed of the combination of $\alpha$, $\beta$, $\gamma$, $\delta$ and $\varepsilon$ subunits, and a variety of subtypes thereof exist. Among these, nicotinic acetylcholine receptors expressed in central nervous systems such as brain, medulla oblongata and spinal cord are called central type nicotinic acetylcholine receptors, which are present as subtypes of $\alpha7$, $\alpha4\beta2$, $\alpha4\beta4$, $\alpha3\beta2$, $\alpha3\beta4$ and the like, and it is known that $\alpha4\beta2$ and $\alpha7$ are main subtypes. For example, $\alpha4\beta2$ subtype is a heteropentamer composed of two $\alpha4$ subunits which are isoforms of $\alpha$ subunit and three $\beta2$ subunits which are isoforms of $\beta$ subunit, which is expressed in cerebral cortex, thalamus and hippocampus; and $\alpha7$ subtype is a homopentamer composed of five $\alpha7$ subunits, which is expressed in cerebral cortex and hippocampus (Non-patent Document 4).

A number of compounds which activate the central type nicotinic acetylcholine receptors have been reported (Patent Document 1 and Non-patent Documents 5 to 9). As a compound which activates $\alpha4\beta2$ and $\alpha7$ subtypes, varenicline(7,8,9,10-tetrahydro-6H-6,10-methanopyrazino[2,3-h][3]benzazepine) has been reported (Non-patent Document 5), and a tartrate thereof is commercially available as a smoking-cessation aid. As a compound which activates $\alpha4\beta2$ subtype, (R)-2-chloro-5-(2-azetidinylmethoxy)pyridine has been reported (Non-patent Document 6), and it has been recently reported that the compound exerts analgesic effect against neuropathic pain of diabetic patients (Non-patent Document 7). As a compound which activates $\alpha7$ subtype, N-(2(S)-(pyridin-3-ylmethyl)-1-azabicyclo[2.2.2]oct-3(R)-yl)-1-benzofuran-2-carboxamide has been reported (Non-patent Document 8), and it has been recently reported that the compound improves cognitive disorders and negative symptoms in schizophrenia in a phase II clinical study (Non-patent Document 9).

Further, acetylcholine and nicotine which activate nicotinic acetylcholine receptors are generally known as substances which induce pruritus in skin (Non-patent Documents 10 and 11).

### PRIOR ART REFERENCES

### PATENT DOCUMENTS

Patent Document 1: WO 2010/132423

### NON-PATENT DOCUMENTS

Non-patent Document 1: Yoshiki Miyachi, "Approaches to Treatment of Pruritus", Sentan Igaku-Sha Ltd., 1996, pp. 22-33
Non-patent Document 2: Sun et al., Nature, 2007, Vol. 448, pp. 700-703

Non-patent Document 3: Liu et al., Nature Neuroscience, 2010, Vol. 13, pp. 1460-1462
Non-patent Document 4: Buccafusco, Molecular Interventions, 2004, Vol. 4, pp. 285-295
Non-patent Document 5: Mihalak et al., Molecular Pharmacology, 2006, Vol. 70, pp. 801-805
Non-patent Document 6: Donnelly-Roberts et al., Journal of Pharmacology and Experimental Therapeutics, 1998, Vol. 285, pp. 777-786
Non-patent Document 7: Rowbotham et al., Pain, 2009, Vol. 146, pp. 245-252
Non-patent Document 8: Hauser et al., Biochemical Pharmacology, 2009, Vol. 78, pp. 803-812
Non-patent Document 9: Targacept Inc., Press release on April 7, 2011, http://www.targacept.com/wt/page/pr_1302179429
Non-patent Document 10: Vogelsang et al., Acta Dermato-Venereologica, 1995, Vol. 75, pp. 434-436
Non-patent Document 11: Smith et al., Skin Pharmacology, 1992, Vol. 5, pp. 69-76

## SUMMARY OF THE INVENTION

### PROBLEMS TO BE SOLVED BY THE INVENTION

However, at present, there are clinically a number of diseases and symptoms in which currently available drugs such as antihistamines fail to exhibit drug effect for pruritus, and the development of an antipruritic which exerts drug effect for pruritus based on a novel action mechanism is strongly demanded.

In view of this, an object of the present invention is to provide an antipruritic effective for pruritus, which exerts drug effect based on a novel action mechanism.

### MEANS FOR SOLVING THE PROBLEMS

The present inventors intensively studied under a situation where the fact that a compound activating a central type nicotinic acetylcholine receptor exhibited an antipruritic effect was not known at all, to find that varenicline commercially available as a smoking-cessation aid can exert a drug effect for pruritus. In addition, the present inventors found that the antipruritic effect observed for varenicline was also observed for a plurality of compounds activating central type nicotinic acetylcholine receptors, thereby completing the present invention.

Accordingly, the present invention provides an antipruritic containing as an effective ingredient a compound which activates a central type nicotinic acetylcholine receptor.

The above-described central type nicotinic acetylcholine receptor is preferably $\alpha7$ subtype or $\alpha4\beta2$ subtype.

### EFFECT OF THE INVENTION

The antipruritic of the present invention exerts an excellent antipruritic effect for pruritus based on an action of activating central type nicotinic acetylcholine receptors, which action is a novel action mechanism. Therefore, the antipruritic of the present invention enables treatment and prophylaxis of pruritus that exhibits resistance to treatment with currently available drugs, and can contribute to improvement of patients' QOL and termination of an itching-scratching cycle.

### BRIEF DESCRIPTION OF THE DRAWINGS

Figure 1 shows the action of a nicotinic acetylcholine receptor antagonist (mecamylamine hydrochloride) on the effect of inhibiting substance P-induced scratching behavior by varenicline tartrate.

### BEST MODE FOR CARRYING OUT THE INVENTION

The antipruritic of the present invention is characterized in that the antipruritic comprises as an effective ingredient a compound which activates a central type nicotinic acetylcholine receptor.

The term "central type nicotinic acetylcholine receptor" refers to a nicotinic acetylcholine receptor which is expressed in central nervous systems including brain, medulla oblongata and spinal cord, and is also referred to as a central nicotinic acetylcholine receptor, neuronal nicotinic acetylcholine receptor, or neuronal type nicotinic acetylcholine receptor.

Examples of the subtype of the central type nicotinic acetylcholine receptor which is activated by the above-described antipruritic include $\alpha7$, $\alpha4\beta2$, $\alpha4\beta4$, $\alpha4\beta2$ and $\alpha3\beta4$, and in view of strength of the antipruritic effects, $\alpha7$ subtype or $\alpha4\beta2$ subtype is preferable, and $\alpha7$ subtype is more preferable.

The phrase "which activates a central type nicotinic acetylcholine receptor" means that a ligand binds to the

receptor, thereby opening the channel of the receptor, and cations are flown into cells from outside of the cells to promote depolarization of plasma membrane, transduction of intracellular signals, or the like.

The "compound which activates a central type nicotinic acetylcholine receptor" may be a compound which directly or indirectly activates the central type nicotinic acetylcholine receptor. Examples thereof include a compound which binds orthosterically or allosterically to the central type nicotinic acetylcholine receptor to activate the receptor; a compound which increases the amount of a released endogenous ligand (for example, endogenous acetylcholine) to be bound to the central type nicotinic acetylcholine receptor or inhibits degradation of the endogenous ligand, thereby increasing the amount of endogenous ligand bound to the central type nicotinic acetylcholine receptor to activate the receptor; and a compound which binds allosterically to the central type nicotinic acetylcholine receptor, thereby increasing responses directly or indirectly which are generated by the endogenous ligand (for example, endogenous acetylcholine) at the orthosteric site of the receptor (which compound may be referred to as a positive allosteric modulator).

Specific examples of the compound which activates a central type nicotinic acetylcholine receptor include varenicline (7,8,9,10-tetrahydro-6H-6,10-methanopyrazino[2,3-h][3]benzazepine), (R)-2-chloro-5-(2-azetidinylmethoxy)pyridine, N-(2(S)-(pyridine-3-ylmethyl)-1-azabicyclo[2.2.2]oct-3(R)-yl)-1-benzofuran-2-carboxamide, N-(1-azabicyclo[2.2.2]oct-3(R)-yl)-4-chlorobenzamide, (R)-7-chloro-N-(quinuclidin-3-yl)benzo[b]thiophene-2-carboxamide, 1,4-diazabicyclo[3.2.2]nonane-4-carboxylic acid 4-bromophenyl ester, 2-(1,4-diazabicyclo[3.2.2]non-4-yl)-5-methyloxazolo[4,5-b]pyridine, 5-(4-morpholinyl)-N-(4-(3-pyridyl)phenyl)pentanamide, cis-2-methyl-5-(6-phenylpyridazin-3-yl)perhydropyrrolo[3,4-c]pyrrole, (-)-N-(1-azabicyclo[2,2,2]oct-3(S)-yl)carbamic acid 1(S)-(2-fluorophenyl)ethyl ester, (R)-spiro(2',3'-dihydro 1-azabicyclo[2.2.2]octane-3,2'-furo[2,3-b]pyridine, 5-(6-(1-azabicyclo[2.2.2]oct-3(R)-yloxy)pyridazin-3-yl)-1H-indole, N-(1-azabicyclo[3.2.1]oct-3(R)-yl)furo[2,3-c]pyridine-5(R)-carboxamide, N-(1-azabicyclo[2.2.2]oct-3(R)-yl)furo[2,3-c]pyridine-5-carboxamide, N-(1-azabicyclo[2.2.2]oct-3(R)-yl)-2,3-dihydro-1,4-benzodioxin-6-carboxamide, N-(5-chloro-2,4-dimethoxyphenyl)-N'-(5-methylisoxazol-3-yl)urea, N-(5-chloro-2-hydroxyphenyl)-N'-(2-chloro-5-(trifluoromethyl)phenyl)urea, 1-(2-(4-fluoro-3-(trifluoromethyl)phenylamino)-4-(4-pyridyl)thiazol-5-yl)methanol, N-(3-(2,2-difluoro-1,3-benzodioxol-5-yl)-5-methyl-2,3-dihydrothiazol-2(Z)-ylidene)-N',N'-dimethylurea, 4-(5-(4-chlorophenyl)-2-methyl-3-propionyl-1H-pyrrol-1-yl)benzenesulfonamide and 3(S)-fluoro-N-(5-(hydioxymethyl)-3-(2,2,3,3-tetrafluoro-2,3-dihydro-1,4-benzodioxin-6-yl)-2,3-dihydrothiazol-2(Z)-ylidene)pyrrolidine-1-carboxamide; and a pharmaceutically acceptable salt thereof.

As the compound which activates a central type nicotinic acetylcholine receptor, preferred is varenicline (7,8,9,10-tetrahydro-6H-6,10-methanopyrazino[2,3-h][3]benzazepine), (R)-2-chloro-5-(2-azetidinylmethoxy)pyridine, N-(2(S)-(pyridine-3-ylmethyl)-1-azabicyclo[2.2.2]oct-3(R)-yl)-1-benzofuran-2-carboxamide, N-(1-azabicyclo[2.2.2]oct-3(R)-yl)-4-chlorobenzamide, (R)-7-chloro-N-(quinuclidine-3-yl)benzo[b]thiophene-2-carboxamide, 1,4-diazabicyclo[3.2.2]nonane-4-carboxylic acid 4-bromophenyl ester, 2-(1,4-diazabicyclo[3.2.2]non-4-yl)-5-methyloxazolo[4,5-b]pyridine, 5-(4-morpholinyl)-N-(4-(3-pyridyl)phenyl)pentanamide, cis-2-methyl-5-(6-phenylpyridazin-3-yl)perhydropyrrolo[3,4-c]pyrrole, (-)-N-(1-azabicyclo[2,2,2]oct-3(S)-yl)carbamic acid 1(S)-(2-fluorophenyl)ethyl ester, N-(1-azabicyclo[2.2.2]oct-3(R)-yl)furo[2,3-c]pyridine-5-carboxamide or N-(1-azabicyclo[2.2.2]oct-3(R)-yl)-2,3-dihydro-1,4-benzodioxin-6-carboxamide; or a pharmaceutically acceptable salt thereof. Varenicline (7,8,9,10-tetrahydro-6H-6,10-methanopyrazino[2,3-h][3]benzazepine) or N-(1-azabicyclo[2.2.2]oct-3(R)-yl)-4-chlorobenzamide; or a pharmaceutically acceptable salt thereof is more preferred.

Examples of the pharmaceutically acceptable salt include inorganic acid salts such as hydrochlorides, sulfates, hydrobromates, or phosphates; and organic acid salts such as formates, acetates, trifluoroacetates, maleates, tartrates, methanesulfonates, benzenesulfonates, toluenesulfonates, or fumarates. In cases where the compound which activates a central type nicotinic acetylcholine receptor is varenicline, the tartrate thereof is preferred.

The compound which activates a central type nicotinic acetylcholine receptor may form, depending on selection of substituents, for example, alkali metal salts such as sodium salts or potassium salts; alkaline earth metal salts such as calcium salts or magnesium salts; organic amine salts such as trimethylamine salts, triethylamine salts, pyridine salts, picoline salts, dicyclohexylamine salts or N,N'-dibenzylethylenediamine salts; or ammonium salts.

Further, the compound which activates a central type nicotinic acetylcholine receptor may be an anhydride or may form a solvate such as a hydrate. Here, as the solvate, a pharmaceutically acceptable solvate is preferred. Although the pharmaceutically acceptable solvate may be a hydrate or non-hydrate, a hydrate is preferred. Examples of solvents constituting the solvate include water, alcohol (for example, methanol, ethanol and n-propanol), dimethylformamide or dimethyl sulfoxide. Further, in cases where crystal polymorphism of the compound which activates a central type nicotinic acetylcholine receptor exists, the compound may be a crystal composed of a single crystal form or may be a mixture of a plurality of crystals having different crystal forms.

Although the compound which activates a central type nicotinic acetylcholine receptor may have an asymmetric carbon depending on the type of substituents and may have optical isomers, all of these optical isomers are included in the compound which activates a central type nicotinic acetylcholine receptor.

The compound which activates a central type nicotinic acetylcholine receptor may be a derivative or prodrug thereof.

The term "prodrug" means one obtained by converting an active form of a drug (which refers to a "drug" corresponding to the prodrug) into an inactive substance by chemical modification for the purpose of improvement of bioavailability, reduction of adverse effects or the like, and means a substance which is metabolized to the active form of the drug after being absorbed into the body to exhibit the effect. That is, the term "prodrug" means any substance which has lower intrinsic activity compared to the active form of the corresponding drug, but once administered into a biological system, undergoes a spontaneous chemical reaction, enzymatically catalyzed reaction or metabolic reaction, resulting in generation of the active form of the drug.

Varenicline(7,8,9,10-tetrahydro-6H-6,10-methanopyrazino[2,3-h][3]benzazepine) and a derivative thereof are described in WO 99/35131 or the like, and exhibit activating effects on mainly $\alpha4\beta2$ and $\alpha7$ subtypes (Mihalak et al., Molecular Pharmacology, 2006, Vol. 70, p. 801). (R)-2-chloro-5-(2-azetidinylmethoxy)pyridine and a derivative thereof are described in WO 98/25920 or the like, and exhibit activating effects on mainly $\alpha4\beta2$ subtype (Donnelly-Roberts et al., Journal of Pharmacology and Experimental Therapeutics, 1998, Vol. 285, p. 777). N-(2(S)-(pyridine-3-ylmethyl)-1-azabicyclo[2.2.2]oct-3(R)-yl)-1-benzofuran-2-carboxamide and a derivative thereof are described in WO 09/018505 or the like, and exhibit activating effects on mainly $\alpha7$ subtype (Hauser et al., Biochemical Pharmacology, 2009, Vol. 78, p. 803). N-(1-azabicyclo[2.2.2]oct-3(R)-yl)-4-chlorobenzamide and a derivative thereof are described in EP A 311724 or the like, and exhibit activating effects on mainly $\alpha7$ subtype (Walker et al., Bioorganic & Medicinal Chemistry, 2006, Vol. 14, p. 8219). (R)-7-chloro-N-(quinuclidin-3-yl)benzo[b]thiophene-2-carboxamide and a derivative thereof are described in WO 03/055878 or the like, and exhibit activating effects on mainly $\alpha7$ subtype (WO 2010/132423). 1,4-diazabicyclo[3.2.2]nonane-4-carboxylic acid 4-bromophenyl ester and a derivative thereof are described in EP A 1231212 or the like, and exhibit activating effects on mainly $\alpha7$ subtype (Biton et al., Neuropsychopharmacology, 2007, Vol. 32, p. 1). 2-(1,4-diazabicyclo[3.2.2]non-4-yl)-5-methyloxazolo[4,5-b]pyridine and a derivative thereof are described in the report by O'Donnell et al. (Journal of Medicinal Chemistry, 2010, Vol. 53, p. 1222) or the like, and exhibit activating effects on mainly $\alpha7$ subtype (O'Donnell et al., Journal of Medicinal Chemistry, 2410, Vol. 53, p. 1222). 5-(4-morpholinyl)-N-(4-(3-pyridyl)phenyl)pentanamide and a derivative thereof are described in WO 06/008133 or the like, and exhibit activating effects on mainly $\alpha7$ subtype (Haydar et al., Bioorganic & Medicinal Chemistry, 2009, Vol. 17, p. 5247). Cis-2-methyl-5-(6-phenylpyridazin-3-yl)perhydropyrrolo[3,4-c]pyrrole and a derivative thereof are described in WO 05/028477 or the like, and exhibit activating effects on mainly $\alpha7$ subtype (Tietje et al., CNS Neuroscience & Therapeutics, 2008, Vol. 14, p. 65). (-)-N-(1-azabicyclo[2,2,2]oct-3(S)-yl)carbamic acid 1(S)-(2-fluorophenyl)ethyl ester and a derivative thereof are described in the report by Jiang et al. (Synthetic Communications, 2009, Vol. 39, p. 2640) or the like, and exhibit activating effects on mainly $\alpha7$ subtype (Feuerbach et al., Neuroscience Letters, 2007, Vol. 416, p. 61). (R)-spiro(2',3'-dihydro-1-azabicyclo[2.2.2]octane-3,2'-furo[2,3-b]pyridine and a derivative thereof are described in WO 99/03859 or the like, and exhibit activating effects on mainly $\alpha7$ subtype (Sydserff et al., Biochemical Pharmacology, 2009, Vol. 78, p .880). 5-(6-(1-azabicyclo[2.2.2]oct-3(R)-yloxy)pyridazin-3-yl)-1H-indole and a derivative thereof are described in WO 06/065233 or the like, and exhibit activating effects on mainly $\alpha7$ subtype (Malysz et al., Journal of Pharmacology and Experimental Therapeutics, 2010, Vol. 334, p. 863). N-(1-azabicyclo[3.2.1]oct-3(R)-yl)furo[2,3-c]pyridine-5(R)-carboxamide and a derivative thereof are described in WO 03/029252 or the like, and exhibit activating effects on mainly $\alpha7$ subtype (Acker et al., Bioorganic & Medicinal Chemistry Letters, 2008, Vol. 18, p. 3611). N-(1-azabicyclo[2.2.2]oct-3(R)-yl)furo[2,3-c]pyridine-5-carboxamide and a derivative thereof are described in WO 02/100857 or the like, and exhibit activating effects on mainly $\alpha7$ subtype (Walker et al., Bioorganic & Medicinal Chemistry, 2006, Vol. 14, p. 8219). N-(1-azabicyclo[2.2.2]oct-3(R)-yl)-2,3-dihydro-1,4-benzodioxin-6-carboxamide and a derivative thereof are described in WO 03/042210 or the like, and exhibit activating effects on mainly $\alpha7$ subtype (Walker et al., Bioorganic & Medicinal Chemistry, 2006, Vol. 14, p. 8219). N-(5-chloro-2,4-dimethoxyphenyl)-N'-(5-methylisoxazol-3-yl)urea and a derivative thereof are described in WO 03/093250 or the like, and exhibit activating effects on mainly $\alpha7$ subtype (Hurst et al., Journal ofNeuroscience, 2005, Vol. 25, p. 4396). N-(5-chloro-2-hydroxyphenyl)-N'-(2-chloro-5-(trifluoromethyl)phenyl)urea and a derivative thereof are described in WO 05/092843 or the like, and exhibit activating effects on mainly $\alpha7$ subtype (Timmermann et al., Journal of Pharmacology and Experimental Therapeutics, 2007, Vol. 323, p. 294). 1-(2-(4-fluoro-3-(trifluoromethyl)phenylamino)-4-(4-pyridyl)thiazol-5-yl)methanol and a derivative thereof are described in WO 07/031440 or the like, and exhibit activating effects on mainly $\alpha7$ subtype (Dinklo et al., Journal of Pharmacology and Experimental Therapeutics, 2011, Vol. 336, p. 560). N-(3-(2,2-difluoro-1,3-benzodioxol-5-yl)-5-methyl-2,3-dihydrothiazol-2(Z)-ylidene)-N',N'-dimethylurea and a derivative thereof are described in WO 08/002956 or the like, and exhibit activating effects on mainly $\alpha7$ subtype (Drug Data Report, 2008, Vol. 30, p. 115). 4-(5-(4-chlorophenyl)-2-methyl-3-propionyl-1H-pyrrol-1-yl)benzenesulfonamide and a derivative thereof are described in WO 08/002974 or the like, and exhibit activating effects on mainly $\alpha7$ subtype (Faghih et al., Journal of Medicinal Chemistry, 2009, Vol. 52, p. 3377). 3(S)-fluoro-N-(5-(hydroxymethyl)-3-(2,2,3,3-tetrafluoro-2,3-dihydro-1,4-benzodioxin-6-yl)-2,3-dihydrothiazol-2(Z)-ylidene)pyrrolidine-1-carboxamide and a derivative thereof are described in WO 08/002956 or the like, and exhibit activating effects on mainly $\alpha7$ subtype (Drug Data Report, 2009, Vol. 31, p. 760).

The compound which activates a central type nicotinic acetylcholine receptor can be produced by known methods. For instance, varenicline(7,8,9,10-tetrahydro-6H-6,10-methanopyrazino[2,3-h][3]benzazepine) and a derivative thereof can be produced by a method disclosed in WO 99/35131 or the like. Varenicline tartrate can also be obtained as an effective ingredient in a formulation such as a tablet. (R)-2-chloro-5-(2-azetidinylmethoxy)pyridine and a derivative thereof can be produced by a method disclosed in WO 98/25920 or the like. N-(2(S)-(pyridin-3-ylmethyl)-1-azabicyclo[2.2.2]oct-3(R)-yl)-1-benzofuran-2-carboxamide and a derivative thereof can be produced by a method disclosed in WO 09/018505 or the like. N-(1-azabicyclo[2.2.2]oct-3(R)-yl)-4-chlorobenzamide and a derivative thereof can be produced by a method disclosed in EP A 311724 or the like. (R)-7-chloro-N-(quinuclidin-3-yl)benzo[b]thiophene-2-carboxamide and a derivative thereof can be produced by a method disclosed in WO 03/055878 or the like. 1,4-diazabicyclo[3.2.2]nonane-4-carboxylic acid 4-bromophenyl ester and a derivative thereof can be produced by a method disclosed in EP A 1231212 or the like. 2-(1,4-diazabicyclo[3.2.2]non-4-yl)-5-methyloxazolo[4,5-b]pyridine and a derivative thereof can be produced by a method disclosed in the report by O'Donnell et al. (Journal of Medicinal Chemistry, 2010, Vol. 53, p. 1222) or the like. 5-(4-morpholinyl)-N-(4-(3-pyridyl)phenyl)pentanamide and a derivative thereof can be produced by a method disclosed in WO 06/008133 or the like. Cis-2-methyl-5-(6-phenylpyridazin-3-yl)perhydropyrrolo[3,4-c]pyrrole and a derivative thereof can be produced by a method disclosed in WO 05/028477 or the like. (-)-N-(1-azabicyclo[2,2,2]oct-3(S)-yl)carbamic acid 1(S)-(2-fluorophenyl)ethyl ester and a derivative thereof can be produced by a method disclosed in the report by Jiang et al. (Synthetic Communications, 2009, Vol. 39, p. 2640) or the like. (R)-spiro(2',3'-dihydro-1-azabicyclo[2.2,2]octane-3,2'-furo[2,-3-b]pyridine and a derivative thereof can be produced by a method disclosed in WO 99/03859 or the like. 5-(6-(1-azabicyclo[2.2.2]oct-3(R)-yloxy)pyridazin-3-yl)-1H-indole and a derivative thereof can be produced by a method disclosed in WO 06/065233 or the like. N-(1-azabicyclo[3.2.1]oct-3(R)-yl)furo[2,3-c]pyridine-5(R)-carboxamide and a derivative thereof can be produced by a method disclosed in WO 03/029252 or the like. N-(1-azabicyclo[2.2.2]oct-3(R)-yl)furo[2,3-c]pyridine-5-carboxamide and a derivative thereof can be produced by a method disclosed in WO 02/100857 or the like. N-(1-azabicyclo [2.2.2]oct-3(R)-yl)-2,3-dihydro-1,4-benzodioxin-6-carboxamide and a derivative thereof can be produced by a method disclosed in WO 03/042210 or the like. N-(5-chloro-2,4-dimethoxyphenyl)-N'-(5-methylisoxazol-3-yl)urea and a derivative thereof can be produced by a method disclosed in WO 03/093250 or the like. N-(5-chloro-2-hydroxyphenyl)-N'-(2-chloro-5-(trifluoromethyl)phenyl)urea and a derivative thereof can be produced by a method disclosed in WO 05/092843 or the like. 1-(2-(4-fluoro-3-(trifluoromethyl)phenylamino)-4-(4-pyridyl)thiazol-5-yl)methanol and a derivative thereof can be produced by a method disclosed in WO 07/031440 or the like. N-(3-(2,2-difluoro-1,3-benzodioxol-5-yl)-5-methyl-2,3-dihydrothiazol-2(Z)-ylidene)-N',N'-dimethylurea and a derivative thereof can be produced by a method disclosed in WO 08/002956 or the like. 4-(5-(4-chlorophenyl)-2-methyl-3-propionyl-1H-pyrrol-1-yl)benzenesulfonamide and a derivative thereof can be produced by a method disclosed in WO 08/002974 or the like. 3(S)-fluoro-N-(5-(hydroxymethyl)-3-(2,2,3,3-tetrafluoro-2,3-dihydro-1,4-benzodioxin-6-yl)-2,3-dihydrothiazol-2(Z)-ylidene)pyrrolidine-1-carboxamide and a derivative thereof can be produced by a method disclosed in WO 08/002956 or the like. Among these compounds, commercially-available ones can also be obtained from a chemical manufacturer or the like.

Pruritus is a sensation which accompanies a desire to scratch and is peculiar to skin, and examples thereof include pruritus caused by skin diseases such as atopic dermatitis, nervous dermatitis, contact dermatitis, seborrheic dermatitis, autosensitization dermatitis, caterpillar dermatitis, asteatosis cutis, senile pruritus cutaneous, insect sting, photosensitivity disorders, urticaria, prurigo, herpes, impetigo, eczema, tinea, lichen, psoriasis, scabies or acne vulgaris; pruritus caused by malignant tumor, diabetes, hepatic diseases, chronic renal diseases, renal failure, hematological disorders, hemodialysis, peritoneal dialysis or multiple sclerosis; and drug-induced or psychogenic pruritus. Although pruritus is divided into pruritus which is mediated by histamine and pruritus which is not mediated by histamine (intractable pruritus), in particular, the antipruritic of the present invention is effective against the pruritus which is not mediated by histamine (intractable pruritus). Examples of the pruritus which is not mediated by histamine (intractable pruritus) include pruritus which is caused by atopic dermatitis, contact dermatitis, asteatosis cutis, senile pruritus, urticaria, psoriasis, malignant tumor, hepatic diseases, chronic renal diseases, renal failure, hematological disorders, hemodialysis, peritoneal dialysis and multiple sclerosis, and exhibits resistant to treatment with antihistamines.

The antipruritic effect of the antipruritic can be evaluated in an *in vivo* experiment system using pruritus model animals, and a pruritus model using murine scratching behavior as an index induced by various pruritogens represented by histamine, chloroquine or substance P is usually employed. For example, substance P-induced scratching behavior using mice described in the document by Togashi et al. (European Journal of Pharmacology, 2002, Vol. 435, p. 259) or the document by Andoh et al. (European Journal of Pharmacology, 2002, Vol. 436, p. 235); or spontaneous scratching behavior using NC/Nga mice described in the document by Takano et al. (European Journal of Pharmacology, 2003, Vol. 471, p. 223) can be used as one of the intractable pruritus models which are not mediated by histamine.

Since the scratching behavior in mice induced by substance P is inhibited by an opioid $\mu$ receptor antagonist, the behavior is not recognized as pain-related response, but as pruritus-related response (Journal of Pharmacology and Experimental Therapeutics, 1998, Vol. 286, p. 1140).

The substance P-induced scratching behavior in mice is not inhibited by tacrolimus as an immunosuppressant (Biological & Pharmaceutical Bulletin, 2008, Vol. 31, p. 752), and indomethacin and diclofenac as antiinflammatory agents. However, leukotriene B4 is participated as a pruritogen and the scratching behavior is inhibited by steroids which

inhibit production thereof (Journal of Investigative Dermatology, 2001, Vol. 117, p. 1621). Therefore, the substance P-induced scratching behavior is induced by acute stimulation of nerves by exogenous substance P and endogenous leukotriene B4, and can be used as a pruritus model which is not mediated by immune responses or inflammatory reactions.

The antipruritic can be used as a pharmaceutical useful in treatment and prophylaxis of pruritus for mammals (for example, mice, rats, hamsters, rabbits, dogs, monkeys, cows, sheep or humans). In clinical use, a free form or salt of the compound which activates a central type nicotinic acetylcholine receptor may be used as it is, or additives such as excipients, stabilizers, preservatives, buffering agents, solubilizing agents, emulsifiers, diluents or isotonic agents may be mixed appropriately. The antipruritic can be produced by a conventional method by appropriately using these pharmaceutical carriers. Examples of administration modes of the antipruritic include oral preparations such as tablets, capsules, granules, powders or syrups; parenteral preparations such as inhalants, injection solutions, suppositories or solutions; and ointments, creams or patches for topical administration. Further, the antipruritic may be prepared as a known sustained-release preparation.

The antipruritic contains as an effective ingredient the compound which activates a central type nicotinic acetylcholine receptor preferably in an amount of 0.001 to 90% by weight, and more preferably in an amount of 0.01 to 70% by weight. Although the dose may be selected depending on the symptoms, age, body weight, sex, administration method and the like, in case of an injection solution, a dose of 0.001 mg to 5 g, and in case of an oral preparation, a dose of 0.01 mg to 10 g, in terms of the effective ingredient, is administered to an adult per day in one time or dividedly in several times.

Examples of pharmaceutically acceptable carriers or diluents of the antipruritic include binders (syrup, gelatin, gum arabic, sorbitol, polyvinyl chloride, tragacanth or the like), excipients (sugar, lactose, corn starch, calcium phosphate, sorbitol, glycine, or the like), and lubricants (magnesium stearate, polyethylene glycol, talc, silica, or the like).

The antipruritic may be formulated or used in combination with other drugs in an appropriate amount in order to complement or enhance the antipruritic effect, or reduce the dose.

Examples of drugs which may be used in combination with the antipruritic include drugs usually used in treatment of the following primary diseases causing pruritus.

Examples of skin diseases causing pruritus include atopic dermatitis, nervous dermatitis, contact dermatitis, seborrheic dermatitis, autosensitization dermatitis, caterpillar dermatitis, asteatosis cutis, senile pruritus, insect sting, photosensitivity disorders, urticaria, prurigo, herpes, impetigo, eczema, tinea, lichen, psoriasis, scabies and acne vulgaris. Examples of other primary diseases include malignant tumor, diabetes, hepatic diseases, chronic renal diseases, renal failure, pregnancy and multiple sclerosis. In addition, the case where pruritus is caused by hemodialysis, peritoneal dialysis or a drug; the case where pruritus is caused by pregnancy or parasitic infection; and psychogenic pruritus are also known.

Examples of drugs used in treatment of atopic dermatitis include topical steroids (betamethasone, beclomethasone, clobetasone, prednisolone or the like), calcineurin inhibiting (immunosuppressing) external preparations (tacrolimus or the like), non-steroid anti-inflammatory external preparations, antihistamines (diphenhydramine, chlorpheniramine, cetirizine, oxatomide, loratadine, or the like), cyclosporin, steroids for oral administration, and moisturizers (urea, Hirudoid, vaseline or the like). Further, examples of drugs used in treatment of multiple sclerosis include corticosteroids (prednisolone, methyl prednisolone or the like), immunosuppressants (methotrexate, azathioprine, cyclophosphamide, cyclosporin A, tacrolimus, mizoribine or the like), interferon formulations (interferon $\alpha$, interferon $\beta$ or the like), sphingosine-1-phosphate receptor modulators (FTY-720), copolymer I, immunoglobulins, T cell receptor vaccine, adhesion molecule inhibitors, TNF $\alpha$ inhibitors, drugs relieving spasticity (tizanidine, eperisone, afloqualone, baclofen, dantrolene or the like), and analgesics (indomethacin, diclofenac or the like).

The present invention will now be described in more detail by way of the following Reference Examples and Examples. However, the present invention is not restricted thereto.

EXAMPLES

(Reference Example 1) Synthesis of (R)-2-Chloro-5-(2-azetidinylmethoxy)pyridine hydrochloride (hereinafter referred to as the compound of Reference Example 1):

[First step]

Synthesis of 2-Chloro-5-((1-(tert-butoxycarbonyl))-2-(R)-azetidinylmethoxy)pyridine:

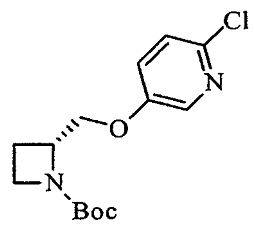

To a solution (2.5 mL) of diisopropyl azodicarboxylate (0.19 mL, 1.0 mmol) in tetrahydrofuran, triphenylphosphine (0.26 g, 1.0 mmol) was added at 0°C, and the resulting mixture was kept at 0°C and stirred. Half an hour later, 1-(tert-butoxycarbonyl)-2-(R)-azetidinylmethanol (0.19 g, 1.0 mmol) and 6-chloropyridin-3-ol (0.13 g, 1.0 mmol) were added thereto, and the resultant was stirred at room temperature. Four hours later, the reaction solution was concentrated and purified by silica gel column chromatography (hexane/ethyl acetate= 95/5 to 70/30) to obtain the title compound (0.27 g; 90%) as a yellow liquid.
$^1$H-NMR (400 MHz, $CDCl_3$)
$\delta$: 1.41 (9H, s), 2.23-2.40 (2H, m), 3.87-3.91 (2H, m), 4.09-4.14 (1H, m), 4.28-4.37 (1H, m), 4.47-4.54 (1H, m), 7.21-7.28 (2H, m), 8.10 (1H, d, J=3.6 Hz).
MS (ESI) $[M+H]^+$
299

[Second step]

Synthesis of (R)-2-Chloro-5-(2-azetidinylmethoxy)pyridine:

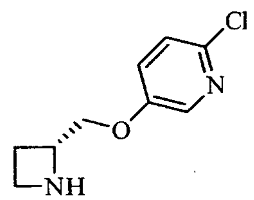

To a solution (10 mL) of 2-chloro-5-((1-(tert-butoxycarbonyl))-2-(R)-azetidinylmethoxy)pyridine (0.27 g, 0.90 mmol) in dichloromethane, trifluoroacetic acid (2.5 mL, 33 mmol) was added, and the resulting mixture was stirred at room temperature. Two hours later, a 1.0 N aqueous sodium hydroxide solution was added to the reaction solution until the pH reached 10, and the resultant was extracted with chloroform. The organic layer was washed with brine, and dried over anhydrous sodium sulfate and concentrated. The obtained crude product was purified by silica gel column chromatography (amine silica gel DM1020, Fuji Silysia Chemical Ltd., chloroform alone to chloroform/methanol = 85/15) to obtain the title compound (0.12 g; 56%) as a yellow liquid.
$^1$H-NMR (400 MHz, $CDCl_3$)
$\delta$: 2.21-2.43 (2H, m), 3.42-3.48 (1H, m), 3.68-3.75 (1H, m), 3.97-4.06 (2H, m), 4.23-4.31 (1H, m), 7.21-7.22 (2H, m), 8.06-8.08 (1H, m).
MS (ESI) $[M+H]^+$
199

[Third step]

Synthesis of Compound of Reference Example 1:

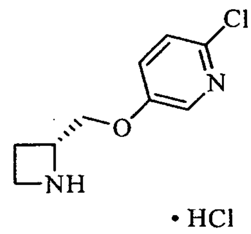

To a solution (1.0 mL) of (R)-2-chloro-5-(2-azetidinylmethoxy)pyridine (0.12 g, 0.61 mmol) in ethyl acetate, a hydrogen chloride-ethyl acetate solution (4.0 M, 0.18 mL, 0.68 mmol) was gently added, and the resulting mixture was stirred at room temperature. Half an hour later, the obtained solid was filtered off, washed with hexane, and dried to obtain the compound of Reference Example 1 (0.10 g; 70%) as a white solid.
$^1$H-NMR (400 MHz, $D_2O$)
δ: 2.63-2.72 (2H, m), 4.02-4.18 (2H, m), 4.40 (2H, d, J=4.0 Hz), 4.90-4.97 (1H, m), 7.46 (1H, d, J=8.8 Hz), 7.56 (1H, dd, J=2.8, 8.8 Hz), 8.12-8.15 (1H, m).
MS (ESI) $[M+H]^+$
199

(Reference Example 2) Synthesis of N-(2(S)-(Pyridin-3-ylmethyl)-1-azabicyclo[2.2.2]oct-3(R)-yl)-1-benzofuran-2-carboxamide (hereinafter referred to as the compound of Reference Example 2):

[First step]

Synthesis of 2-(3-Pyridinyl)methylene-1-azabicyclo[2.2.2]octan-3-one:

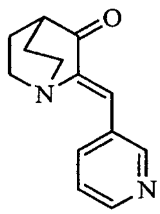

To 3-quinuclidinone hydrochloride (6.0 g, 37 mmol), a solution (36 mL) of potassium hydroxide (2.3 g, 41 mmol) in methanol and 3-pyridinealdehyde (4.4 g, 41 mmol) were added, and the resulting mixture was stirred at room temperature. Sixteen hours later, distilled water (30 mL) was added to the reaction solution, and the resultant was cooled to 4°C and left to stand for 16 hours. The obtained solid was filtered off, washed with distilled water, and dried to obtain the title compound (7.6 g; 96%) as a yellow solid.
$^1$H-NMR (400 MHz, $CDCl_3$)
δ: 2.00-2.08 (4H, m), 2.64-2.68 (1H, m), 2.94-3.04 (2H, m), 3.13-3.22 (2H, m), 6.98 (1H, s), 7.28-7.32 (1H, m), 8.44-8.50 (1H, m), 8.51-8.55 (1H, m), 9.04 (1H, d, J=2.0 Hz).
MS (ESI) $[M+H]^+$
215

[Second step]

Synthesis of 2-((3-Pyridinyl)methyl)-1-azabicyclo[2.2.2]octan-3-one:

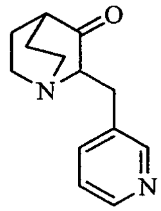

To a solution (80 mL) of 2-(3-pyridinyl)methylene-1-azabicyclo[2.2.2]octan-3-one (7.6 g, 36 mmol) in methanol, Pd/C (10% wet, 0.64 g, 0.60 mmol) and hydrochloric acid (6.0 N, 9.0 mL, 54 mmol) were added, and the resulting mixture was stirred under hydrogen atmosphere at room temperature. Sixteen hours later, the reaction solution was filtered with Celite and the filtrate was concentrated. A 2.0 N aqueous sodium hydroxide solution was added to the obtained crude product until the pH reached 10, and the resultant was extracted with chloroform. The organic layer was washed with brine and then dried over anhydrous sodium sulfate and concentrated. The obtained crude product was purified by silica gel column chromatography (hexane/ethyl acetate = 90/10 to 59/41, chloroform alone to chloroform/methanol = 78/22) to obtain the title compound (5.4 g; 71 %) as a colorless liquid.
$^1$H-NMR (400 MHz, $CDCl_3$)
δ: 1.92-2.10 (4H, m), 2.47-2.51 (1H, m), 2.73-2.92(3H, m), 3.05-3.24(3H, m), 3.29-3.35 (1H, m), 7.20-7.24 (1H, m), 7.57-7.62 (1H, m), 8.44-8.48 (1H, m), 8.52 (1H, d, J=2.0 Hz).
MS (ESI) $[M+H]^+$
217

[Third step]

Synthesis of Compound of Reference Example 2:

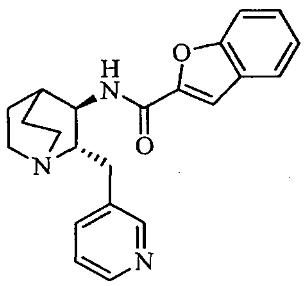

To a solution (34 mL) of 2-((3-pyridinyl)methyl)-1-azabicyclo[2.2.2]octan-3-one (5.0 g, 23 mmol) in methanol, zinc dichloride (630 mg, 4.6 mmol) was added, and the resulting mixture was stirred at room temperature. Half an hour later, ammonium formate (17 g, 280 mmol) was added thereto, and the resultant was stirred at room temperature. One hour later, sodium cyanoborohydride (2.9 g, 46 mmol) was added thereto, and the resultant was stirred at room temperature. Sixteen hours later, the mixture was heated to 50°C and stirred. Three hours later, distilled water and a 5.0 N aqueous sodium hydroxide solution were added to the reaction solution, and the resultant was neutralized and then extracted with chloroform. The organic layer was washed with brine, then dried over anhydrous sodium sulfate and concentrated. The obtained crude product was purified by silica gel column chromatography (amine silica gel DM1020, Fuji Silysia Chemical Ltd., chloroform alone to chloroform/methanol = 89/11). To the obtained purified product, methanol (16 mL) and (+)-di-p-toluoyl-(D)-tartaric acid (4.0 g, 10 mmol) were added, and the resultant was stirred at 90°C. One hour later, the reaction solution was concentrated, methanol (4.0 mL) was added thereto, and the resultant was heated to 90°C to dissolve the concentrate. The reaction solution was slowly cooled to room temperature over 1 hour, to 5°C over 4 hours, and subsequently to 0°C, and left to stand for 16 hours. The obtained precipitate was filtered off and recrystallized using methanol (3.0 mL). To the obtained solid, a 2.0 N aqueous sodium hydroxide solution (3.0 mL) and chloroform (3.0 mL) were added, and the resultant was extracted with chloroform. Organic layers were washed with brine, then dried over anhydrous sodium sulfate and concentrated. The obtained colorless liquid was dissolved in dichloromethane (2.0 mL), the resulting mixture was added to a solution (2.0 mL) of o-(benzotriazol-1-yl)-N,N,N',N'-tetramethyluronium hexafluorophosphate(0.60 g, 1.6 mmol), triethylamine (0.73 mL, 1.6 mmol), and benzofuran-2-carboxylic acid (0.17 g, 1.1 mmol) in dichloromethane, and the resultant was stirred at room temperature. Sixteen hours later,

distilled water was added thereto and the resultant was extracted with chloroform. The organic layer was washed with brine, and then dried over anhydrous sodium sulfate and concentrated. The obtained crude product was purified by silica gel column chromatography (chloroform alone to chloroform/methanol = 99/1) to obtain the compound of Reference Example 2 (0.21 g; 3%) as a white solid.
$^1$H-NMR (400 MHz, $CDCl_3$)
δ: 1.50-1.59 (1H, m), 1.66-1.84(3H, m), 2.00-2.06 (1H, m), 2.72-2.82 (2H, m), 2.92-2.99 (4H, m), 3.05-3.14 (1H, m), 3.94-3.99 (1H, m), 6.55-6.60 (1H, m), 7.12-7.16 (1H, m), 7.29-7.32 (1H, m), 7.40-7.45 (2H, m), 7.51 (1H, d, J=8.4 Hz), 7.56-7.60 (1H, m), 7.67 (1H, d, J=8.4 Hz), 8.35 (1H, dd, J=1.6, 4.4 Hz), 8.51 (1H, d, J=2.4 Hz).
MS (ESI) $[M+H]^+$
362

(Reference Example 3) Synthesis of (R)-7-Chloro-N-(quinuclidin-3-yl)benzo[b]thiophene-2-carboxamide hydrochloride (hereinafter referred to as the compound of Reference Example 3):

[First step]

Synthesis of 7-Chloro-1-benzothiophene-2-carboxylic acid:

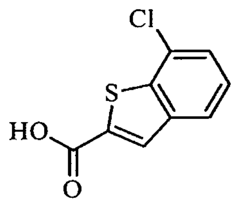

To an aqueous solution (10 mL) of potassium hydroxide (1.3 g, 22 mmol), 2-mercaptoacetic acid (0.70 mL, 10 mmol) and 2,3-dichlorobenzaldehyde (1.8 g, 10 mmol) were added, and the resulting mixture was stirred at 120°C. Two hours later, the reaction solution was cooled to room temperature, and after adding distilled water until precipitated solid was dissolved, diethyl ether was added thereto. The reaction solution was separated into an organic layer and water layer, and after adding 1.0 N hydrochloric acid to the water layer until the pH reached 5, the water layer was extracted with ethyl acetate. The organic layer was washed with brine, dried over anhydrous sodium sulfate and concentrated to obtain the title compound (1.7 g; 82%) as a colorless liquid.
$^1$H-NMR (400 MHz, DMSO-$d_6$)
δ: 7.48-7.54 (1H, m), 7.62-7.70 (1H, m), 7.98-8.04 (1H, m), 8.16-8.22 (1H, m).
MS (ESI) $[M+H]^+$
213

[Second step]

Synthesis of (R)-7-Chloro-N-(quinuclidine-3-yl)benzo[b]thiophene-2-carboxamide:

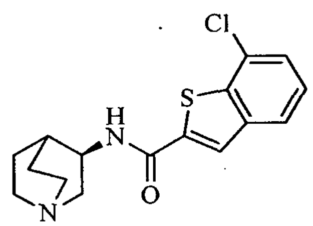

To a solution (10 mL) of 7-chloro-1-benzothiophene-2-carboxylic acid (210 mg, 1.0 mmol) in chloroform, o-(benzotriazol-1-yl)-N,N,N',N'-tetramethyluronium hexafluorophosphate (570 mg, 1.5 mmol) and diisopropylethylamine (0.70 mL, 4.0 mmol) were added. Thereafter, (R)-quinuclidine-3-amine hydrochloride (200 mg, 1.0 mmol) was added thereto, and the resulting mixture was stirred at room temperature. Sixteen hours later, distilled water and 1.0 N aqueous sodium

hydroxide solution were added thereto, and the resultant was extracted with chloroform. The organic layer was washed with brine, then dried over anhydrous sodium sulfate and concentrated. The obtained crude product was purified by silica gel column chromatography (amine silica gel DM1020, Fuji Silysia Chemical Ltd., chloroform alone to chloroform/methanol = 90/10) to obtain the title compound (170 mg; 53%) as a white solid.
$^1$H-NMR (400 MHz, DMSO-$d_6$)
δ: 1.22-1.38 (1H, m), 1.53-1.62 (2H, m), 1.75-1.82 (2H, m), 2.63-2.73 (4H, m), 2.84-2.94 (1H, m), 3.07-3.18 (1H, m), 3.90-4.00 (1H, m), 7.49 (1H, dd, J=7.6, 8.0 Hz), 7.59 (1H, d, J=7.6 Hz), 7.96 (1H, d, J=8.0 Hz), 8.31 (1H, s), 8.62-8.66 (1H, m).
MS (ESI) $[M+H]^+$
321

[Third step]

Synthesis of Compound of Reference Example 3:

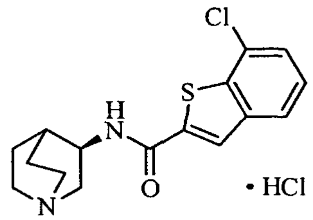

To a solution (2.0 mL) of (R)-7-chloro-N-(quinuclidin-3-yl)benzo[b]thiophene-2-carboxamide (170 mg, 0.53 mmol) in ethyl acetate, hydrogen chloride-ethyl acetate solution (4.0 M, 0.20 mL, 0.80 mmol) was added, and the resulting mixture was stirred at room temperature. Ten minutes later, the obtained solid was filtered off, washed with ethyl acetate and hexane, and dried to obtain the compound of Reference Example 3 (170 mg; 90%) as a white solid.
$^1$H-NMR (400 MHz, DMSO-$d_6$)
δ: 1.70-1.78 (1H, m), 1.86-1.94 (2H, m), 2.10-2.19 (2H, m), 3.18-3.35 (5H, m), 3.63-3.72 (1H, m), 4.27-4.36 (1H, m), 7.50 (1H, d, J=7.6, 8.0 Hz), 7.61 (1H, d, J=7.6 Hz), 7.98 (1H, d, J=8.0 Hz), 8.38 (1H, s), 9.07-9.10 (1H, m), 9.80-9.85 (1H, m).
MS (ESI) $[M+H]^+$
321

(Reference Example 4) Synthesis of 1,4-Diazabicyclo[3.2.2]nonane-4-carboxylic acid 4-bromophenyl ester hydrochloride (hereinafter referred to as the compound of Reference Example 4):

[First step]

Synthesis of 1,4-Diazabicyclo[3.2.2]nonane-4-carboxylic acid 4-bromophenyl ester:

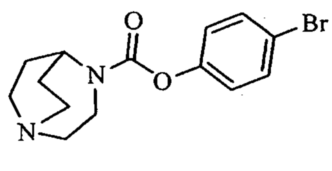

To a solution (12 mL) of 1,4-diazabicyclo[3.2.2]nonane dihydrochloride (230 mg, 1.2 mmol) in dichloromethane, diisopropylethylamine (0.40 mL, 2.4 mmol) was added, and the resulting mixture was stirred at room temperature for 1 hour. To a solution (12 mL) oftriphosgene (110 mg, 0.38 mmol) in dichloromethane, 4-bromophenol (200 mg, 1.2 mmol) and a solution (12 mL) of diisopropylethylamine (0.20 mL, 1.2 mmol) in tetrahydrofuran were gently added, and the resultant was stirred at room temperature. One hour later, the previously prepared solution of 1,4-diazabicyclo[3.2.2] nonane dihydrochloride was gently added to this solution, and the resultant was stirred at room temperature. Four hours later, water was added to the reaction solution, and the resultant was extracted with chloroform. The organic layer was washed with brine, then dried over anhydrous sodium sulfate and concentrated. The obtained crude product was purified

by silica gel column chromatography (hexane/ethyl acetate = 95/5 to 70/30, chloroform alone to chloroform/methanol = 90/10) to obtain the title compound (0.24 g; 65%) as a colorless liquid.
$^1$H-NMR (400 MHz, $CDCl_3$)
δ: 1.68-1.81 (2H, m), 2.00-2.14 (2H, m), 2.95-3.19(6H, m), 3.72-3.85 (2H, m), 4.34-4.45 (1H, m), 6.98-7.06 (2H, m), 7.44-7.50 (2H, m).
MS (ESI) $[M+H]^+$
326

[Second step]

Synthesis of Compound of Reference Example 4:

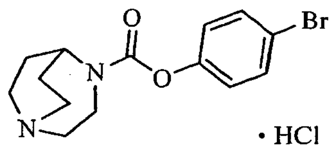

To a solution (4.0 mL) of 1,4-diazabicyclo[3.2.2]nonane-4-carboxylic acid 4-bromophenyl ester (250 mg, 0.75 mmol) in ethyl acetate, a hydrogen chloride-ethyl acetate solution (4.0 M, 0.28 mL, 1.1 mmol) was added, and the resulting mixture was stirred at room temperature. Ten minutes later, the obtained solid was filtered off, washed with ethyl acetate and hexane, and dried to obtain the compound of Reference Example 4 (240 mg; 88%) as a white solid.
$^1$H-NMR (400 MHz, DMSO-$d_6$)
δ: 1.95-2.26 (4H, m), 3.32-3.47(6H, m), 3.81-3.85 (1H, m), 3.94-4.00 (1H, m), 4.35-4.56 (1H, m), 7.14 (2H, d, J=8.8 Hz), 7.59 (2H, d, J=8.8 Hz), 10.2-11.0 (1H, m).
MS (ESI) $[M+H]^+$
326

(Reference Example 5) Synthesis of 2-(1,4-Diazabicyclo[3.2.2]non-4-yl)-5-methyloxazolo[4,5-b]pyridine hydrochloride (hereinafter referred to as the compound of Reference Example 5):

[First step]

Synthesis of 2-Amino-6-methyl pyridin-3-ol:

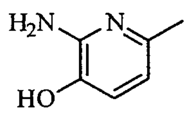

To a solution (20 mL) of 6-methyl-2-nitropyridin-3-ol (1.6 g, 10 mmol) in ethanol, Pd/C (10% wet, 0.40 g, 0.38 mmol) and acetic acid (0.050 mL, 0.87 mmol) were added, and the resulting mixture was stirred under hydrogen atmosphere at room temperature. Six hours later, the reaction solution was filtered with Celite and the filtrate was concentrated. The obtained crude product was purified by silica gel column chromatography (amine silica gel DM1020, Fuji Silysia Chemical Ltd., chloroform alone to chloroform/methanol = 88/12) to obtain the title compound (1.2 g; 93%) as a yellow liquid.
$^1$H-NMR (400 MHz, $CDCl_3$)
δ: 2.32(3H, s), 6.38 (1H, d, J=8.0 Hz), 6.82-6.86 (1H, m).

[Second step]

Synthesis of 5-Methyloxazolo[4,5-b]pyridine-2-thiol:

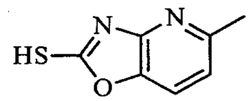

To a solution (25 mL) of 2-amino-6-methylpyridin-3-ol (1.2 g, 9.7 mmol) in ethanol, potassium ethyl xanthate (3.1 g, 19 mmol) was added, and the resulting mixture was stirred at 80°C. Four hours later, the reaction solution was concentrated, distilled water was added, and acetic acid was added to the resultant until the pH reached 5. The obtained solid was filtered off, washed with distilled water, and dried to obtain the title compound (1.2 g; 77%) as a white solid.
$^1$H-NMR (400 MHz, $CDCl_3$)
δ: 2.45(3H, s), 7.03-7.07 (1H, m), 7.64-7.70 (1H, m).

[Third step]

Synthesis of 5-Methyl-2-(methylthio)oxazolo[4,5-b]pyridine:

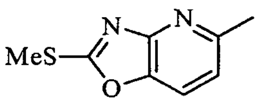

To a solution (20 mL) of 5-methyloxazolo[4,5-b]pyridine-2-thiol (1.2 g, 7.5 mmol) in N,N-dimethylformamide, potassium carbonate (1.0 g, 7.5 mmol) and methyl iodide (0.56 mL, 9.0 mmol) were added, and the resulting mixture was stirred at room temperature. Three hours later, distilled water was added thereto and the resultant was extracted with ethyl acetate. The organic layer was washed with distilled water and brine, then dried over anhydrous sodium sulfate and concentrated. The obtained crude product was purified by silica gel column chromatography (hexane/ethyl acetate = 88/12 to 65/35, chloroform alone to chloroform/methanol = 98/2) to obtain the title compound (1.2 g; 90%) as a white solid.
$^1$H-NMR (400 MHz, DMSO-$d_6$)
δ: 2.53(3H, s), 2.77(3H, s), 7.18 (1H, d, J=8.0 Hz), 7.94 (1H, d, J=8.0 Hz).

[Fourth step]

Synthesis of 2-(1,4-Diazabicyclo[3.2.2]non-4-yl)-5-methyloxazolo[4,5-b]pyridine:

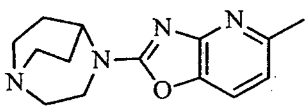

To 1,4-diazabicyclo[3.2.2]nonane dihydrochloride (900 mg, 4.5 mmol), an aqueous sodium hydroxide solution (1.0 N, 11 mL, 11 mmol) was added, and the resulting mixture was stirred at room temperature. Two hours later, the reaction solution was extracted with a chloroform-methanol mixed solution (9:1), and the organic layer was washed with distilled water and brine, dried over anhydrous sodium sulfate and concentrated. To the obtained crude product, isopropyl alcohol (2.7 mL) and 5-methyl-2-(methylthio)oxazolo[4,5-b]pyridine (300 mg, 1.7 mmol) were added, and the resultant was stirred at 100°C. Twenty two hours later, the obtained crude product was purified by silica gel column chromatography (amine silica gel DM1020, Fuji Silysia Chemical Ltd., chloroform alone to chloroform/methanol = 95/5) to obtain the title compound (320 mg; 74%) as a white solid.
$^1$H-NMR (400 MHz, $CD_3OD$)
δ: 1.74-1.85 (2H, m), 2.10-2.21 (2H, m), 2.53(3H, s), 2.95-3.06 (2H, m), 3.10-3.19 (4H, m), 3.95 (2H, t, J=6.0 Hz), 4.53-4.57 (1H, m), 6.72 (1H, d, J=7.6 Hz), 7.28 (1H, d, J=7.6 Hz).
MS (ESI) $[M+H]^+$
259

[Fifth step]

Synthesis of Compound of Reference Example 5:

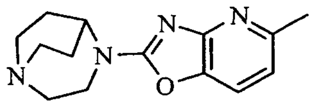

• HCl

To a solution (1.0 mL) of 2-(1,4-diazabicyclo[3.2.2]non-4-yl)-5-methyloxazolo[4,5-b]pyridine (320 mg, 1.2 mmol) in ethyl acetate, a hydrogen chloride-ethyl acetate solution (4.0 M, 0.47 mL, 1.9 mmol) was added, and the resulting mixture was stirred at room temperature. Half an hour later, the obtained white solid was filtered off, washed with ethyl acetate and hexane, and dried to obtain the compound of Reference Example 5 (290 mg; 80%) as a white solid. $^1$H-NMR (400 MHz, $CDCl_3$)
δ: 2.05-2.17 (2H, m), 2.22-2.34 (2H, m), 2.49(3H, s), 3.35-3.44 (4H, m), 3.48-3.54 (2H, m), 4.09-4.12 (2H, m), 4.59-4.65 (1H, m), 6.99 (1H, d, J=8.0 Hz), 7.82-7.87 (1H, m), 11.4-11.5 (1H, m).
MS (ESI) $[M+H]^+$
259

(Reference Example 6) Synthesis of 5-(4-Morpholinyl)-N-(4-(3-pyridyl)phenyl)pentanamide hydrochloride (hereinafter referred to as the compound of Reference Example 6):

[First step]

Synthesis of 5-Bromo-N-(4-bromophenyl)pentanamide:

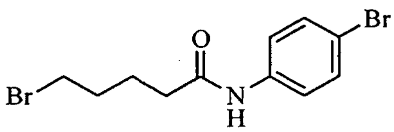

To a solution (60 mL) of 4-bromoaniline (2.8 g, 17 mmol) in dichloromethane, triethylamine (2.3 mL, 17 mmol) was added, and 5-bromopentanoyl chloride (2.4 mL, 18 mmol) was then gently added thereto at 0°C, and the resulting mixture was stirred while keeping the temperature at 0°C. Three hours later, the reaction solution was heated to room temperature, 0.40 N aqueous sodium carbonate solution was added thereto and the resultant was extracted with ethyl acetate. The organic layer was washed with brine, dried over anhydrous sodium sulfate and concentrated to obtain the title compound (5.5 g; 99%) as a white solid.
$^1$H-NMR (400 MHz, $CDCl_3$)
δ: 1.85-2.00 (4H, m), 2.40 (2H, t, J=6.8 Hz), 3.45 (2H, t, J=6.0 Hz), 7.08-7.12 (1H, m), 7.39-7.43 (4H, m).
MS (ESI) $[M+H]^+$
336

[Second step]

Synthesis of 5-Morpholin-4-yl-pentanoic acid(4-bromophenyl)amide:

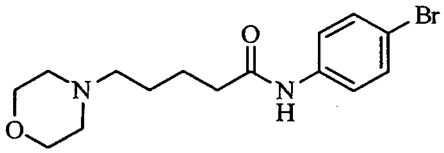

To a solution (60 mL) of 5-bromo-N-(4-bromophenyl)pentanamide (2.7 g, 8.1 mmol) in 2-butanone, sodium iodide (1.2 g, 8.1 mmol) and morpholine (0.78 mL, 8.9 mmol) were added, and the resulting mixture was stirred at 70°C. Six hours later, 1.0 N aqueous sodium hydroxide solution was added thereto, and the resulting mixture was concentrated and extracted with ethyl acetate. The organic layer was washed with brine, then dried over anhydrous sodium sulfate and concentrated. The obtained crude product was purified by silica gel column chromatography (hexane alone to hexane/ethyl acetate = 80/20, chloroform alone to chloroform/methanol = 95/5) to obtain the title compound (2.3 g; 83%) as a white solid.
$^1$H-NMR (400 MHz, $CDCl_3$) δ: 1.53-1.62 (2H, m), 1.72-1.80 (2H, m), 2.35-2.45(8H, m), 3.67-3.72 (4H, m), 7.16-7.21 (1H, m), 7.39-7.42 (4H, m).
MS (ESI) $[M+H]^+$
342

[Third step]

Synthesis of 5-(4-Morpholinyl)-N-(4-(3-pyridyl)phenyl)pentanamide:

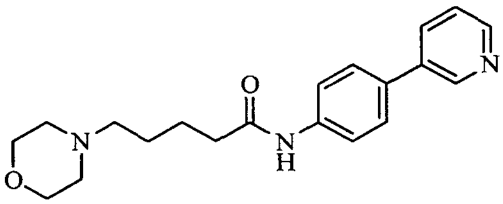

To a solution (8.0 mL) of 5-morpholin-4-yl-pentanoic acid(4-bromophenyl)amide (500 mg, 1.5 mmol) in acetonitrile, pyridine-3-boronic acid (200 mg, 1.6 mmol) and aqueous sodium carbonate solution (0.4 M, 8.0 mL) were added. Thereafter, tetrakis(triphenylphosphine)palladium (85 mg, 0.073 mmol) was added thereto, and the resulting mixture was stirred under microwave irradiation at 90°C. Twenty minutes later, the reaction solution was extracted with ethyl acetate. The organic layer was washed with brine, then dried over anhydrous sodium sulfate and concentrated. The obtained crude product was purified by silica gel column chromatography (chloroform alone to chloroform/methanol = 80/20) to obtain the title compound (350 mg; 71 %) as a white solid.
$^1$H-NMR (400 MHz, DMSO-$d_6$)
δ: 1.40-1.50 (2H, m), 1.54-1.65 (2H, m), 2.24-2.36(8H, m), 3.52-3.67 (4H, m), 7.42-7.47 (1H, m), 7.65-7.73 (4H, m), 8.00-8.05 (1H, m), 8.49-8.54 (1H, m), 8.83-8.87 (1H, m), 9.98-10.0 (1H, m).
MS (ESI) $[M+H]^+$
340

[Fourth step]

Synthesis of Compound of Reference Example 6:

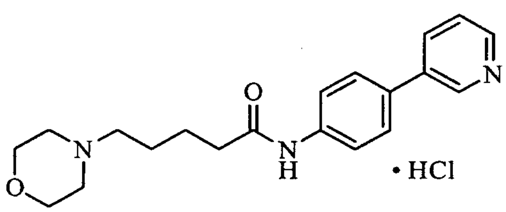

To a solution (2.0 mL) of 5-(4-morpholinyl)-N-(4-(3-pyridyl)phenyl)pentanamide (360 mg, 1.0 mmol) in methanol, hydrochloric acid (1.0 N, 1.0 mL, 1.0 mmol) was added, and the resulting mixture was stirred at room temperature. Ten minutes later, a solid was filtered off, washed with ethyl acetate and hexane, and dried to obtain the compound of Reference Example 6 (340 mg; 87%) as a white solid.
$^1$H-NMR (400 MHz, DMSO-$d_6$)
δ: 1.59-1.78 (4H, m), 2.39-2.45 (2H, m), 2.96-3.14 (4H, m), 3.33-3.42 (2H, m), 3.71-3.81 (2H, m), 3.88-3.96 (2H, m), 7.78-7.82 (4H, m), 7.86-7.94 (1H, m), 8.55-8.65 (1H, m), 8.71-8.76 (1H, m), 9.10-9.14 (1H, m), 10.3-10.4 (1H, m), 10.7-10.8 (1H, m).
MS (ESI) $[M+H]^+$
340

(Reference Example 7) Synthesis of cis-2-methyl-5-(6-phenylpyridazin-3-yl)perhydropyrrolo[3,4-c]pyrrole fumarate (hereinafter referred to as the compound of Reference Example 7):

[First step]

Synthesis of Tert-butyl cis-5-(6-phenylpyridazine-3-yl)-hexahydro-pyrrolo[3,4-c]-pyrrole-2(1H)-carboxylate:

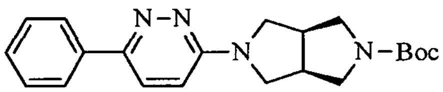

To a solution (5.0 mL) of 3-chloro-6-phenylpyridazine (0.90 g, 4.7 mmol) in dimethyl sulfoxide, tert-butyl cis-hexahydropyrrolo[3,4-c]pyrrole-2(1H)-carboxylate (1.0 g, 4.7 mmol) and diisopropylethylamine (2.9 mL, 16 mmol) were added, and the resulting mixture was stirred at 105°C. Twenty one hours later, the reaction solution was cooled to 40°C, water was added thereto, and the resultant was stirred. One hour later, the obtained solid was filtered off, washed with water and diethyl ether, and dried to obtain the title compound (0.98 g; 57%) as a brown solid.
$^1$H-NMR (400 MHz, $CD_3OD$)
δ: 1.46 (9H, s), 3.07-3.16 (2H, m), 3.30-3.35 (2H, m), 3.46-3.52 (2H, m), 3.62-3.72 (2H, m), 3.77-3.84 (2H, m), 7.00-7.05 (1H, m), 7.37-7.43 (1H, m), 7.43-7.50 (2H, m), 7.81-7.86 (1H, m), 7.89-7.94 (2H, m).
MS (ESI) $[M+H]^+$
367

[Second step]

Synthesis of cis-2-methyl-5-(6-phenylpyridazin-3-yl)perhydropyrrolo[3,4-c]pyrrole:

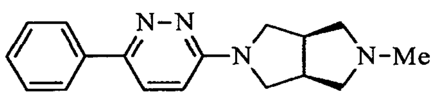

To an aqueous formic acid solution (2.5 mL) of tert-butyl cis-5-(6-phenylpyridazin-3-yl)-hexahydro-pyrrolo[3,4-c]-pyrrole-2(1H)-carboxylate (0.50 g, 1.4 mmol), formalin (30%, 0.089 mL, 1.5 mmol) was added, and the resulting mixture was stirred at 100°C. One hour later, the reaction solution was cooled to room temperature and concentrated, and sodium bicarbonate water was then added thereto to neutralize the concentrate. After extracting the resultant with chloroform, the organic layer was washed with brine, then dried over anhydrous sodium sulfate and concentrated. The obtained crude product was purified by silica gel column chromatography (amine silica gel DM1020, Fuji Silysia Chemical Ltd., chloroform alone to chloroform/methanol = 95/5) to obtain the title compound (0.29 g; 76%) as a white solid.
$^1$H-NMR (400 MHz, $CD_3OD$)
δ: 2.28(3H, s), 2.44-2.50 (2H, m), 2.75-2.80 (2H, m), 3.00-3.08 (2H, m), 3.45-3.52 (2H, m), 3.62-3.68 (2H, m), 7.01 (1H, d, J=9.6 Hz), 7.31-7.44(3H, m), 7.78 (1H, d, J=9.6 Hz), 7.85 (2H, d, J=8.0 Hz).
MS (ESI) $[M+H]^+$
281

[Third step]

Synthesis of Compound of Reference Example 7:

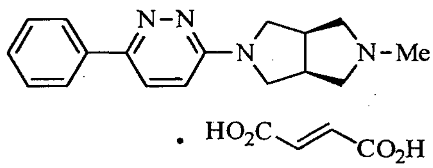

To a methanol-diethyl ether mixed solution (1/10, 10 mL) of cis-2-methyl-5-(6-phenylpyridazin-3-yl)perhydropyrrolo[3,4-c]pyrrole (0.29 g, 1.0 mmol), a methanol-diethyl ether mixed solution (1/10, 10 mL) of fumaric acid (0.12 g, 1.0 mmol) was added, and the resulting mixture was stirred at room temperature. One hour later, a solid was filtered off, and the obtained white solid was washed with diethyl ether and hexane and dried to obtain the compound of Reference Example 7 (360 mg; 88%) as a white solid.
$^1$H-NMR (400 MHz, DMSO-$d_6$)
δ: 2.32(3H, s), 2.58-2.64 (2H, m), 2.68-2.76 (2H, m), 2.95-3.05 (2H, m), 3.43-3.49 (2H, m), 3.64-3.71 (2H, m), 6.56 (2H, s), 7.01 (1H, d, J=9.6 Hz), 7.35-7.50(3H, m), 7.91 (1H, d, J=9.6 Hz), 8.00 (2H, d, J=7.2 Hz).
MS (ESI) $[M+H]^+$
281

(Reference Example 8) Synthesis of (-)-N-(1-Azabicyclo[2,2,2]oct-3(S)-yl)carbamic acid 1 (S)-(2-fluorophenyl)ethyl ester (hereinafter referred to as the compound of Reference Example 8):

[First step]

Synthesis of (S)-1-(2-Fluorophenyl)ethanol:

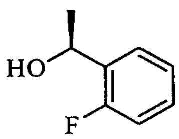

To a solution (60 mL) of (R)-methyl oxazaborolidine (0.32 g, 1.2 mmol) in tert-butyl alcohol, N,N-diethylaniline borane (2.6 g, 16 mmol) was added, and a solution (150 mL) of 1-(2-fluorophenyl)ethanone (2.0 g, 14 mmol) in tert-butyl alcohol was then added thereto at 45°C, and the resulting mixture was stirred. One hour later, the reaction solution was cooled to room temperature, methanol was added thereto and the resultant was concentrated. After 1.0 N hydrochloric acid was added thereto, the resultant was extracted with ethyl acetate, and the organic layer was washed with brine, then dried over anhydrous sodium sulfate and concentrated. The obtained crude product was purified by silica gel column chromatography (hexane alone to hexane/ethyl acetate = 85/15) to obtain the title compound (1.9 g; 92%) as a colorless liquid.
$^1$H-NMR (400 MHz, $CDCl_3$)
δ: 1.52(3H, d, J=6.8 Hz), 5.17-5.24 (1H, m), 6.98-7.05 (1H, m), 7.15 (1H, ddd, J=1.2, 7.6, 7.6 Hz), 7.21-7.28 (1H, m), 7.49 (1H, ddd, J=1.6, 7.6, 7.6 Hz).

[Second step]

Synthesis of Imidazole-1-carboxylic acid (S)-1-(2-fluorophenyl)ethyl ester:

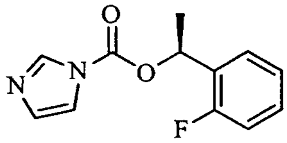

To a solution (16 mL) of 1,1'-carbonyldiimidazole (3.3 g, 20 mmol) in tetrahydrofuran, a solution (4.0 mL) of (S)-1-(2-fluorophenyl)ethanol (1.9 g, 13 mmol) in tetrahydrofuran was gently added at 50°C, and the resulting mixture was stirred. Four hours later, distilled water was added thereto and the resultant was extracted with ethyl acetate. The organic layer was washed with brine, then dried over anhydrous sodium sulfate and concentrated. The obtained crude product was purified by silica gel column chromatography (hexane/ethyl acetate= 90/10 to 70/30) to obtain the title compound (3.1 g; 99%) as a colorless liquid.
$^1$H-NMR (400 MHz, $CDCl_3$)
δ: 1.75(3H, d, J=6.4 Hz), 6.33 (1H, q, J=6.4 Hz), 7.06-7.13 (2H, m), 7.18 (1H, ddd, J=1.2, 7.2, 7.6 Hz), 7.31-7.46(3H, m), 8.16 (1H, s).
MS (ESI) $[M+H]^+$
235

[Third step]

Synthesis of Compound of Reference Example 8:

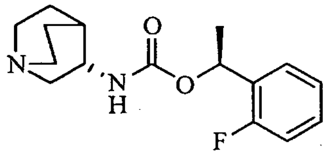

To a solution (14 mL) of 3-aminoquinuclidine dihydrochloride (1.4 g, 7.0 mmol) in tetrahydrofuran, n-butyl lithium (2.6 M hexane solution, 5.2 mL, 14 mmol) was gently added at 0°C. The resulting mixture was stirred at room temperature for 1 hour, and then imidazole-1-carboxylic acid (S)-1-(2-fluorophenyl)ethyl ester (1.5 g, 6.4 mmol) was gently added thereto at 0°C. After stirring the resultant at room temperature for 1 hour, and then at 55°C for 2 hours, distilled water was added thereto and the resultant was extracted with ethyl acetate. The organic layer was washed with brine, then dried over anhydrous sodium sulfate and concentrated. The obtained crude product was purified by silica gel column chromatography (amine silica gel DM1020, Fuji Silysia Chemical Ltd., chloroform alone to chloroform/methanol = 98/2) to obtain the compound of Reference Example 8 (480 mg; 26%) as a white solid.
$^1$H-NMR (400 MHz, $CDCl_3$)
δ: 1.36-1.50 (1H, m), 1.55(3H, d, J=6.4 Hz), 1.53-1.65(3H, m), 1.83-1.90 (1H, m), 2.44-2.52 (1H, m), 2.69-2.85 (4H, m), 3.28-3.38 (1H, m), 3.66-3.74 (1H, m), 4.84-4.94 (1H, m), 6.01-6.08 (1H, m), 7.00-7.06 (1H, m), 7.13 (1H, dd, J=6.8, 7.6 Hz), 7.22-7.29 (1H, m), 7.37 (1H, dd, J=5.2, 6.8 Hz).
MS (ESI) $[M+H]^+$
293

(Reference Example 9) Synthesis of N-(1-Azabicyclo[2.2.2]oct-3(R)-yl)furo[2,3-c]pyridine-5-carboxamide dihydrochloride(hereinafter referred to as the compound of Reference Example 9):

[First step]

Synthesis of N-(1-Azabicyclo[2.2.2]oct-3(R)-yl)furo[2,3-c]pyridine-5-carboxamide:

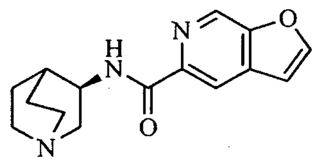

To a solution (10 mL) of furo[2,3-c]pyridine-5-carboxylic acid (0.16 g, 1.0 mmol) in chloroform, o-(benzotriazol-1-yl)-N,N,N',N'-tetramethyluronium hexafluorophosphate (0.57 g, 1.5 mmol), diisopropylethylamine (0.70 mL, 4.0 mmol), and (R)-quinuclidine-3-amine hydrochloride (0.20 g, 1.0 mmol) were added, and the resulting mixture was stirred at room temperature. Sixteen hours later, distilled water was added thereto, and the resultant was then extracted with chloroform. The organic layer was washed with brine, then dried over anhydrous sodium sulfate and concentrated. The obtained crude product was purified by silica gel column chromatography (amine silica gel DM1020, Fuji Silysia Chemical Ltd., chloroform alone to chloroform/methanol = 98/2) to obtain the title compound (0.18 mg; 66%) as a colorless liquid.
$^1$H-NMR (400 MHz, $CDCl_3$)
δ: 1.40-1.55 (1H, m), 1.65-1.74 (2H, m), 1.80-1.90 (1H, m), 2.02-2.06 (1H, m), 2.63-2.70 (1H, m), 2.75-3.00 (4H, m), 3.39-3.46 (1H, m), 4.12-4.20 (1H, m), 6.89-6.92 (1H, m), 7.80 (1H, d, J=2.0 Hz) 8.25-8.35 (1H, m), 8.46-8.49 (1H, m), 8.75-8.78 (1H, m).
MS (ESI) $[M+H]^+$
272

[Second step]

Synthesis of Compound of Reference Example 9:

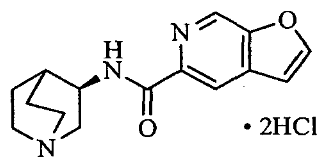

To a solution (2.0 mL) of N-(1-azabicyclo[2.2.2]oct-3(R)-yl)furo[2,3-c]pyridine-5-carboxamide (180 mg, 0.66 mmol) in ethyl acetate, hydrogen chloride-ethyl acetate solution (4.0 M, 0.50 mL, 2.0 mmol) was added, and the resulting mixture was stirred at room temperature. Ten minutes later, the obtained solid was filtered off, washed with ethyl acetate and hexane, and dried to obtain the compound of Reference Example 9 (190 mg; 84%) as a white solid.
$^1$H-NMR (400 MHz, $D_2O$)
δ: 1.76-2.18 (4H, m), 2.26-2.32 (1H, m), 3.15-3.35 (5H, m), 3.68-3.76 (1H, m), 4.35-4.40 (1H, m), 7.01-7.05 (1H, m), 8.00-8.04 (1H, m), 8.32-8.34 (1H, m), 8.80-8.82 (1H, m).
MS (ESI) $[M+H]^+$
272

(Reference Example 10) Synthesis of N-(1-Azabicyclo[2.2.2]oct-3(R)-yl)-2,3-dihydro-1,4-benzodioxin-6-carboxamide hydrochloride (hereinafter referred to as the compound of Reference Example 10):

[First step]

Synthesis of N-(1-Azabicyclo[2.2.2]oct-3R)-yl)-2,3-dihydro-1,4-benzodioxin-6-carboxamide:

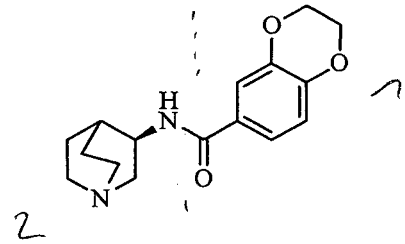

To a solution (10 mL) of 2,3-dihydrobenzo[b][1,4]dioxin-6-carboxylic acid 1 (0.18 g, 1.0 mmol) in chloroform, o-(benzotriazol-1-yl)-N,N, N',N'-tetramethyluronium hexafluorophosphate (0.57 g, 1.5 mmol), diisopropylethylamine (0.70 mL, 4.0 mmol), and (R)-quinuclidine-3-amine hydrochloride 2 (0.20 g, 1.0 mmol) were added, and the resulting mixture was stirred at room temperature. Sixteen hours later, distilled water was added thereto, and the resultant was then extracted with chloroform. The organic layer was washed with brine, then dried over anhydrous sodium sulfate and concentrated. The obtained crude product was purified by silica gel column chromatography (amine silica gel DM1020, Fuji Silysia Chemical Ltd., chloroform alone to chloroform/methanol = 98/2) to obtain the title compound (0.22 mg; 76%) as a colorless liquid.
$^1$H-NMR (400 MHz, $CDCl_3$)
δ: 1.44-1.55 (1H, m), 1.65-1.76(3H, m), 1.98-2.03 (1H, m), 2.51-2.58 (1H, m), 2.75-2.90 (4H, m), 3.37-3.46 (1H, m), 4.05-4.14 (1H, m), 4.25-4.35 (4H, m), 6.08-6.18 (1H, m), 6.86-6.90 (1H, m), 7.24-7.31 (2H, m).
MS (ESI) $[M+H]^+$
289

[Second step]

Synthesis of Compound of Reference Example 10:

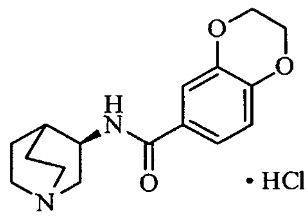

To a solution (2.0 mL) of N-(1-azabicyclo[2.2.2]oct-3(R)-yl)-2,3-dihydro-1,4-benzodioxin-6-carboxamide (220 mg, 0.76 mmol) in ethyl acetate, a hydrogen chloride-ethyl acetate solution (4.0 M, 0.28 mL, 1.1 mmol) was added, and the resulting mixture was stirred at room temperature. Ten minutes later, the obtained solid was filtered off, washed with ethyl acetate and hexane, and dried to obtain the compound of Reference Example 10 (110 mg; 43%) as a white solid.
$^1$H-NMR (400 MHz, DMSO-$d_6$)
δ: 1.64-1.74 (1H, m), 1.82-1.91 (2H, m), 1.97-2.16 (2H, m), 3.10-3.35 (5H, m), 3.55-3.63 (1H, m), 4.22-4.30 (5H, m), 6.91-6.95 (1H, m), 7.40-7.46 (2H, m), 8.42-8.45 (1H, m), 10.2-10.3 (1H, m).
MS (ESI) $[M+H]^+$
289

(Example 1) Inhibitory Effect on Substance P-Induced Scratching Behavior by Compound Activating Central Type Nicotinic Acetylcholine Receptor:

Substance P-induced scratching behavior in mice which is an intractable pruritus model was induced by the method described in a known document (Togashi et al., European Journal of Pharmacology, 2002, Vol. 435, p. 259 and the like). Further, evaluation of the scratching behavior was objectively carried out by automatic detection using MicroAct (NeuroScience, Inc.) based on the method described in a known document (Hashimoto et al., Allergology International, 2004, Vol. 53, p. 349).
More particularly, at least five days before the evaluation of drug effect, neodymium magnets (diameter: 1 mm, length: 3 mm) coated with Parafilm were subcutaneously inserted into the insteps of both hind limbs of 5 to 7 week-old male ICR mouse (Japan SLC, Inc.) under isoflurane anesthesia. One or two days before the day of the evaluation of

drug effect, the hair of the rostral part of the back of the mouse was shaved with a hair clipper under isoflurane anesthesia. At least one hour before the start of the measurement of the number of scratching behavior, each mouse (6 to 8 weeks old) was individually placed in a chamber for measurement (diameter: 11 cm, height: 18 cm) for acclimation. After the acclimation, substance P (5 mmol/L) or phosphate-buffered saline (hereinafter referred to as PBS) as a solvent thereof was intradermally administered (0.05 mL/site) into the rostral part of the back, and the measurement of the number of scratching behavior was started immediately after the administration. The number of scratching behavior was recorded by amplifying electric current induced by the movement of the magnets inserted in the hind limbs in a round coil surrounding the chamber for measurement. The measurement was carried out under unattended environment, and the evaluation of the drug effect was carried out by using the number of scratching behavior occurred for 15 minutes after the start of the measurement as an index.

A test compound or a solvent thereof was administered in an amount of 10 mL/kg 30 to 60 minutes before the start of the measurement of the number of scratching behavior. Varenicline tartrate (Tocris Bioscience) was dissolved in PBS and intraperitoneally administered in a dose of 1, 3 or 10 mg/kg 60 minutes before the start of the measurement of the number of scratching behavior. N-(1-azabicyclo[2.2.2]oct-3(R)-yl)-4-chlorobenzamide hydrochloride (PNU-282987) hydrate (Sigma-Aldrich) was dissolved in PBS and intraperitoneally administered in a dose of 0.3 or 1 mg/kg 45 minutes before the start of the measurement of the number of scratching behavior. The compound of Reference Example 1 was dissolved in PBS and intraperitoneally administered in a dose of 0.1 or 0.3 mg/kg 30 minutes before the start of the measurement of the number of scratching behavior. The compound of Reference Example 2 was dissolved in PBS and intraperitoneally administered in a dose of 1, 3, or 10 mg/kg 45 minutes before the start of the measurement of the number of scratching behavior. The compound of Reference Example 3 was dissolved in distilled water and orally administered in a dose of 1, 3, or 10 mg/kg 30 minutes before the start of the measurement of the number of scratching behavior. The compound of Reference Example 4 was dissolved in PBS and intraperitoneally administered in a dose of 1, 3, or 10 mg/kg 45 minutes before the start of the measurement of the number of scratching behavior. The compound of Reference Example 5 was dissolved in PBS and intraperitoneally administered in a dose of 1, 3, or 10 mg/kg 30 minutes before the start of the measurement of the number of scratching behavior. The compound of Reference Example 6 was dissolved in PBS and intraperitoneally administered in a dose of 1, 3, or 10 mg/kg, 30 minutes before the start of the measurement of the number of scratching behavior. The compound of Reference Example 7 was dissolved in PBS and intraperitoneally administered in a dose of 0.3, 1, or 3 mg/kg 45 minutes before the start of the measurement of the number of scratching behavior. The compound of Reference Example 8 was suspended in 0.5% methyl cellulose and orally administered in a dose of 10 or 30 mg/kg 60 minutes before the start of the measurement of the number of scratching behavior. The compound of Reference Example 9 was dissolved in PBS and intraperitoneally administered in a dose of 0.1, 0.3, or 1 mg/kg 45 minutes before the start of the measurement of the number of scratching behavior. The compound of Reference Example 10 was dissolved in PBS and intraperitoneally administered in a dose of 0.1, 0.3, or 1 mg/kg 45 minutes before the start of the measurement of the number of scratching behavior.

A group in which the solvent alone was administered (test compound: 0 mg/kg, substance P: 0 nmol/site) was designated as "non-induced control group; a group in which substance P was administered, whereas a test compound was not administered (test compound: 0 mg/kg, substance P: 250 nmol/site) was designated as "induced control group"; and a group in which substance P and a test compound were administered was designated as " test compound-administered group".

An inhibition rate (%) of the scratching behavior by the test compound was calculated by the following equation:

$$\text{Inhibition rate (\%)} = [1-(A-C)/(B-C)] \times 100$$

wherein, A, B, and C represent the mean of the number of scratching behavior in the test compound-administered group, induced control group, and non-induced control group respectively.

With regard to statistical processing, Dunnett's test was performed as a test of the test compound-administered group as compared with the induced control group. A level of significance was set to 5% (two-tailed).

Table 1 and Table 2 show the effect of each test compound on the number of scratching behavior. The symbol "*" in the tables indicates statistical significance as compared with the induced control group (*p<0.05, Dunnett's test).

[Table 1]

| Test compound (administration route) | Dose of test compound (mg/kg) | Substance P (nmol/site) | n | Number of scratching behavior (means ± standard error) | Inhibition rate (%) |
|---|---|---|---|---|---|
| Varenicline tartrate (i.p.) | 0 | 0 | 5 | 18±4 | - |
| | 0 | 250 | 8 | 118±14 | - |
| | 1 | | 8 | 103±19 | 15 |
| | 3 | | 8 | 54±7* | 64 |
| | 10 | | 8 | 17±3* | 101 |
| PNU-282987 hydrate (i.p.) | 0 | 0 | 3 | 17±10 | - |
| | 0 | 250 | 5 | 127±27 | - |
| | 0.3 | | 5 | 81±9 | 42 |
| | 1 | | 5 | 48±8* | 72 |
| Compound of Reference Example 1 (i·p.) | 0 | 0 | 5 | 28±9 | - |
| | 0 | 250 | 8 | 132±21 | - |
| | 0.1 | | 8 | 21±8* | 107 |
| | 0.3 | | 8 | 6±2* | 121 |
| Compound of Reference Example 2 (i·p.) | 0 | 0 | 5 | 41±8 | - |
| | 0 | 250 | 8 | 131±25 | - |
| | 1 | | 8 | 67±10* | 71 |
| | 3 | | 8 | 97±14 | 38 |
| | 10 | | 8 | 75±8* | 62 |
| Compound of Reference Example 3 (p.o.) | 0 | 0 | 5 | 21±5 | - |
| | 0 | 250 | 8 | 94±24 | - |
| | 1 | | 8 | 71±14 | 32 |
| | 3 | | 8 | 57±13 | 51 |
| | 10 | | 8 | 36±8* | 79 |
| Compound of Reference Example 4 (i.p.) | 0 | 0 | 5 | 22±6 | - |
| | 0 | 250 | 7 | 121±14 | - |
| | 1 | | 8 | 89±12 | 32 |
| | 3 | | 8 | 59±14* | 63 |
| | 10 | | 8 | 42±6* | 80 |

[Table 2]

| Test compound (administration route) | Dose of test compound (mg/kg) | Substance P (nmol/site) | n | Number of scratching behavior (means ± standard error) | Inhibition rate (%) |
|---|---|---|---|---|---|
| Compound of Reference Example 5 (i.p.) | 0 | 0 | 5 | 11±5 | - |
| | 0 | 250 | 8 | 106±12 | - |
| | 1 | | 8 | 82±6 | 25 |
| | 3 | | 8 | 62±11* | 46 |
| | 10 | | 8 | 50±12* | 59 |
| Compound of Reference Example 6 (i.p.) | 0 | 0 | 5 | 39±16 | - |
| | 0 | 250 | 8 | 120±21 | - |
| | 1 | | 8 | 94±15 | 32 |
| | 3 | | 8 | 78±10 | 52 |
| | 10 | | 8 | 36±5* | 104 |
| Compound of Reference Example 7 (i.p.) | 0 | 0 | 5 | 12±5 | - |
| | 0 | 250 | 7 | 130±18 | - |
| | 0.3 | | 8 | 101±20 | 25 |
| | I | | 8 | 37±8* | 79 |
| | 3 | | 8 | 52±9* | 66 |
| Compound of Reference Example 8 (p.o.) | 0 | 0 | 5 | 24±5 | - |
| | 0 | 250 | 8 | 139±14 | - |
| | 10 | | 8 | 92±17 | 41 |
| | 30 | | 8 | 83±19 | 49 |
| Compound of Reference Example 9 (i.p.) | 0 | 0 | 5 | 20±3 | - |
| | 0 | 250 | 8 | 119±21 | - |
| | 0.1 | | 7 | 109±21 | 10 |
| | 0.3 | | 8 | 76±18 | 43 |
| | 1 | | 8 | 50±10* | 70 |
| Compound of Reference Example 10 (i.p.) | 0 | 0 | 5 | 22±9 | - |
| | 0 | 250 | 8 | 134±24 | - |
| | 0.1 | | 8 | 131±18 | 3 |
| | 0.3 | | 8 | 115±19 | 17 |
| | 1 | | 8 | 69±10* | 58 |

From these results, it is apparent that the compound which activates a central type nicotinic acetylcholine receptor prominently inhibits the substance P-induced scratching behavior which is known as an intractable pruritus model and has an excellent antipruritic effect.

(Example 2) Effect of Nicotinic Acetylcholine Receptor Antagonists on Effect of Inhibiting Substance P-Induced Scratching Behavior by Varenicline

The induction and measurement of the number of substance P-induced scratching behavior were carried out by the same method as described in Example 1.

Varenicline tartrate (0.3 mg/mL, Tocris Bioscience), mecamylamine hydrochloride (0.3 mg/mL, Tocris Bioscience), a mixed solution of varenicline tartrate and mecamylamine hydrochloride (both 0.3 mg/mL), or PBS which was a solvent thereof, was intraperitoneally administered in an amount of 10 mL/kg 60 minutes before the start of the measurement of the number of scratching behavior.

With regard to statistical processing, Student's t-test was performed when variances were homogeneity by F-test, and Aspin-Welch test was performed when variances were heterogeneity. A level of significance was set to 5% (two-tailed).

The results of the evaluation are shown in Fig. 1. The vertical axis indicates the number of scratching behavior occurred for 15 minutes (mean ± standard error, n= 5 to 8). The horizontal axis indicates the non-induced control group (test compound: 0 mg/kg, substance P: 0 nmol/site), induced control group (test compound: 0 mg/kg, substance P: 250 nmol/site), varenicline tartrate-administered group (varenicline tartrate: 3 mg/kg, substance P: 250 nmol/site), mecamylamine hydrochloride-administered group (mecamylamine hydrochloride: 3 mg/kg, substance P: 250 nmol/site), and varenicline tartrate + mecamylamine hydrochloride-administered group (varenicline tartrate: 3 mg/kg, mecamylamine hydrochloride: 3 mg/kg, substance P: 250 nmol/site). The symbol "*" in the figure indicates statistical significance as compared with the induced control group (*$p<0.05$, Aspin-Welch test), and the symbol "#" indicates statistical significance as compared with the varenicline tartrate administered-group (#$p<0.05$, Aspin-Welch test).

When varenicline tartrate was used in combination with mecamylamine hydrochloride which was a nicotinic acetylcholine receptor antagonist, the effect of inhibiting the substance P-induced scratching behavior by varenicline tartrate completely disappeared. From these results, it is apparent that the effect of inhibiting scratching behavior by the compound which activates a central type nicotinic acetylcholine receptor results from the specific activation of the nicotinic acetylcholine receptor.

The substance P-induced scratching behavior is induced by acute stimulation of nerves by exogenous substance P and endogenous leukotriene B4 and is thought to be a pruritus model which is not mediated by immune responses or inflammatory reactions (Andoh et al., European Journal of Pharmacology, 1998, Vol. 353, p. 93; Andoh et al., Journal of Investigative Dermatology, 2001, Vol. 117, p. 1621). The single administration of the compound activating a central type nicotinic acetylcholine receptor 30 to 60 minutes before the induction of the substance P-induced scratching behavior inhibited the scratching behavior, which implies that the activation of the central type nicotinic acetylcholine receptor directly inhibits conduction and/or transmission of impulses by pruritic stimuli.

INDUSTRIAL APPLICABILITY

The present invention can be used as an antipruritic in the medical field.

## Claims

1. An antipruritic comprising as an effective ingredient a compound which activates a central type nicotinic acetylcholine receptor.

2. The antipruritic according to claim 1, wherein said central type nicotinic acetylcholine receptor is $\alpha7$ subtype.

3. The antipruritic according to claim 1, wherein said central type nicotinic acetylcholine receptor is $\alpha4\beta2$ subtype.

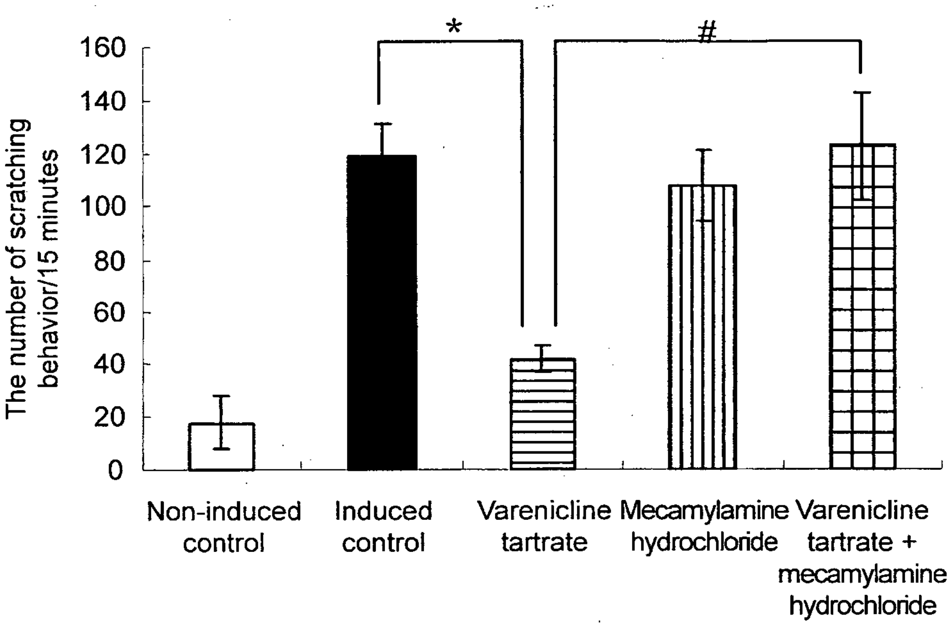
*
#
160
140
120
100
80
60
40
20
0
The number of scratching behavior/15 minutes
Non-induced control
Induced control
Varenicline tartrate
Mecamylamine hydrochloride
Varenicline tartrate + mecamylamine hydrochloride

Fig.1

**INTERNATIONAL SEARCH REPORT**

International application No.
PCT/JP2012/066675

A. CLASSIFICATION OF SUBJECT MATTER
*A61K45/00*(2006.01)i, *A61K31/439*(2006.01)i, *A61K31/444*(2006.01)i, *A61K31/5377*(2006.01)i, *A61P17/04*(2006.01)i, *A61P43/00*(2006.01)i, *A61K31/4427*(2006.01)n, *A61K31/501*(2006.01)n, *A61K31/551*(2006.01)n

According to International Patent Classification (IPC) or to both national classification and IPC

B. FIELDS SEARCHED

Minimum documentation searched (classification system followed by classification symbols)
A61K45/00, A61K31/439, A61K31/444, A61K31/5377, A61P17/04, A61P43/00, A61K31/4427, A61K31/501, A61K31/551

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2012 |
| Kokai Jitsuyo Shinan Koho | 1971-2012 | Toroku Jitsuyo Shinan Koho | 1994-2012 |

Electronic data base consulted during the international search (name of data base and, where practicable, search terms used)
CA/MEDLINE/EMBASE/BIOSIS(STN), JSTPlus/JMEDPlus/JST7580(JDreamII)

C. DOCUMENTS CONSIDERED TO BE RELEVANT

| Category* | Citation of document, with indication, where appropriate, of the relevant passages | Relevant to claim No. |
|---|---|---|
| Y | Rollema H. et al., Varenicline has antidepressant-like activity in the forced swim test and augments sertraline's effect., European Journal of Pharmacology, 2009, Vol.605, No.1-3, pages 114 to 116, ISSN: 0014-2999 | 1-3 |
| Y | Mihalak K.B. et al., Varenicline is a partial agonist at alpha4beta2 and a full agonist at alpha7 neuronal nicotinic receptors., Molecular Pharmacology, 2006, Vol.70, No.3, pages 801 to 805, ISSN: 0026-895X | 1-3 |
| Y | Mayo M.J. et al., Sertraline as a first-line treatment for cholestatic pruritus., Hepatology, 2007, Vol.45, No.3, pages 666 to 674, ISSN: 0270-9139 | 1-3 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 August, 2012 (07.08.12) | 21 August, 2012 (21.08.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2012/066675

C (Continuation). DOCUMENTS CONSIDERED TO BE RELEVANT

| Category* | Citation of document, with indication, where appropriate, of the relevant passages | Relevant to claim No. |
|---|---|---|
| Y | Makoto HASHIRO, "Hifu Shikkan Chiryo no Point, Antidepressants for dermatologists", Rinsho Hifuka, vol.61, no.5, 2007, pages 119 to 122, ISSN: 0021-4973 | 1-3 |
| Y | Steensma D. et al., Selective serotonin reuptake inhibitors can relieve intractable pruritus associated with polycythemia vera., International Journal of Hematology. Supplement, 2002, Vol.76, No.Supplement 1, page 97, P259, ISSN: 0917-1258 | 1-3 |
| Y | Tey H.L., Targeted treatment of pruritus: a look into the future., British Journal of Dermatology, 2011.05.30, [online], doi: 10.1111/j.1365-2133.2011.10217.x., Retrieved from the internet: <URL:http://onlinelibrary.wiley.com/doi/10.1111/j.1365-2133.2011.10217.x/abstract> (Vol.165, No.1, pages 5 to 17, ISSN: 0007-0953) | 1-3 |

Form PCT/ISA/210 (continuation of second sheet) (July 2009)

INTERNATIONAL SEARCH REPORT

International application No.
PCT/JP2012/066675

Subject to be covered by this search:

Claims 1-3 relate to an antipruritic agent comprising, as an active ingredient, a substance that is defined by a desired property "a compound capable of activating a central nervous system nicotinic acetylcholine receptor".

Claims 1-3 include all of substances each having the property within their scopes. However, those substances which are disclosed in the meaning within PCT Article 5 are just some of the claimed compounds. Therefore, it cannot be considered that these claims are supported by the disclosure of the description in the meaning within PCT Article 6.

Further, with respect to the "compound capable of activating a central nervous system nicotinic acetylcholine receptor", even though the common technical knowledge at the time of filing the present application is taken into consideration, it is impossible to specify the scope of the compounds each having the property. Therefore, claims 1-3 do not comply with the requirement of clarity under PCT Article 6, either.

Such being the case, the search was carried out on the relationship between the activation of central nervous system nicotinic acetylcholine receptors and the prevention of pruritus, and also carried out on antipruritic agents each comprising a compound specifically described in the description as an active ingredient.

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2010132423 A
- WO 9935131 A
- WO 9825920 A
- WO 09018505 A
- EP 311724 A
- WO 03055878 A
- EP 1231212 A
- WO 06008133 A
- WO 05028477 A
- WO 9903859 A
- WO 06065233 A
- WO 03029252 A
- WO 02100857 A
- WO 03042210 A
- WO 03093250 A
- WO 05092843 A
- WO 07031440 A
- WO 08002956 A
- WO 08002974 A


### Non-patent literature cited in the description


- **YOSHIKI MIYACHI.** Approaches to Treatment of Pruritus. Sentan Igaku-Sha Ltd, 1996, 22-33
- **SUN et al.** *Nature,* 2007, vol. 448, 700-703
- **LIU et al.** *Nature Neuroscience,* 2010, vol. 13, 1460-1462
- **BUCCAFUSCO.** *Molecular Interventions,* 2004, vol. 4, 285-295
- **MIHALAK et al.** *Molecular Pharmacology,* 2006, vol. 70, 801-805
- **DONNELLY-ROBERTS et al.** *Journal of Pharmacology and Experimental Therapeutics,* 1998, vol. 285, 777-786
- **ROWBOTHAM et al.** *Pain,* 2009, vol. 146, 245-252
- **HAUSER et al.** *Biochemical Pharmacology,* 2009, vol. 78, 803-812
- *Press release,* 07 April 2011
- **VOGELSANG et al.** *Acta Dermato-Venereologica,* 1995, vol. 75, 434-436
- **SMITH et al.** *Skin Pharmacology,* 1992, vol. 5, 69-76
- **MIHALAK et al.** *Molecular Pharmacology,* 2006, vol. 70, 801
- **DONNELLY-ROBERTS et al.** *Journal of Pharmacology and Experimental Therapeutics,* 1998, vol. 285, 777
- **HAUSER et al.** *Biochemical Pharmacology,* 2009, vol. 78, 803
- **WALKER et al.** *Bioorganic & Medicinal Chemistry,* 2006, vol. 14, 8219
- **BITON et al.** *Neuropsychopharmacology,* 2007, vol. 32, 1
- **O'DONNELL et al.** *Journal of Medicinal Chemistry,* 2010, vol. 53, 1222
- **O'DONNELL et al.** *Journal of Medicinal Chemistry,* vol. 53 (2410), 1222
- **HAYDAR et al.** *Bioorganic & Medicinal Chemistry,* 2009, vol. 17, 5247
- **TIETJE et al.** *CNS Neuroscience & Therapeutics,* 2008, vol. 14, 65
- **JIANG et al.** *Synthetic Communications,* 2009, vol. 39, 2640
- **FEUERBACH et al.** *Neuroscience Letters,* 2007, vol. 416, 61
- **SYDSERFF et al.** *Biochemical Pharmacology,* 2009, vol. 78, 880
- **MALYSZ et al.** *Journal of Pharmacology and Experimental Therapeutics,* 2010, vol. 334, 863
- **ACKER et al.** *Bioorganic & Medicinal Chemistry Letters,* 2008, vol. 18, 3611
- **HURST et al.** *Journal ofNeuroscience,* 2005, vol. 25, 4396
- **TIMMERMANN et al.** *Journal of Pharmacology and Experimental Therapeutics,* 2007, vol. 323, 294
- **DINKLO et al.** *Journal of Pharmacology and Experimental Therapeutics,* 2011, vol. 336, 560
- *Drug Data Report,* 2008, vol. 30, 115
- **FAGHIH et al.** *Journal of Medicinal Chemistry,* 2009, vol. 52, 3377
- *Drug Data Report,* 2009, vol. 31, 760
- **TOGASHI et al.** *European Journal of Pharmacology,* 2002, vol. 435, 259
- **ANDOH et al.** *European Journal of Pharmacology,* 2002, vol. 436, 235
- **TAKANO et al.** *European Journal of Pharmacology,* 2003, vol. 471, 223
- *Journal of Pharmacology and Experimental Therapeutics,* 1998, vol. 286, 1140

- *Biological & Pharmaceutical Bulletin,* 2008, vol. 31, 752
- *Journal of Investigative Dermatology,* 2001, vol. 117, 1621
- **HASHIMOTO et al.** *Allergology International,* 2004, vol. 53, 349
- **ANDOH et al.** *European Journal of Pharmacology,* 1998, vol. 353, 93
- **ANDOH et al.** *Journal of Investigative Dermatology,* 2001, vol. 117, 1621